# EUROPEAN PATENT SPECIFICATION

(11) **EP 2 841 092 B1**
(45) Date of publication and mention of the grant of the patent: **06.12.2023**
(21) Application number: 13749329.2
(22) Date of filing: 19.02.2013
(51) Int. Cl.: A61K 38/47, A61K 31/16, A61P 11/12, A61K 9/00, A61P 11/00, A61P 31/14, A61P 31/16, A61P 43/00

(54) **COMPOUNDS AND COMPOSITIONS FOR USE IN A METHOD OF TREATMENT OF PARAINFLUENZA VIRUS**
VERBINDUNGEN UND ZUSAMMENSETZUNGEN ZUR ANWENDUNG IN EINEM VERFAHREN ZUR BEHANDLUNG EINES PARAINFLUENZAVIRUS
COMPOSÉS ET COMPOSITIONS POUR UTILISATION DANS UNE MÉTHODE DE TRAITEMENT DU VIRUS PARAINFLUENZA

(30) Priority: 17.02.2012 US 201261600545 P; 16.11.2012 US 201261727627 P
(43) Date of publication of application: 04.03.2015
(73) Proprietor: Ansun Biopharma, Inc., San Diego, CA 92121 (US)
(72) Inventor: MOSS, Ron, Encinitas, California 92024 (US); LI, Tiejun, Princeton, NJ 08540 (US)
(74) Representative: Lau, Sarah Jane
(86) International application number: PCT/US2013/026754
(87) International publication number: WO 2013/123521

(56) References cited:
- US-A1- 2004 081 624
- US-A1- 2007 190 163
- US-A1- 2011 171 132
- ANNE MOSCONA ET AL: "A Recombinant Sialidase Fusion Protein Effectively Inhibits Human Parainfluenza Viral Infection In Vitro and In Vivo", THE JOURNAL OF INFECTIOUS DISEASES, vol. 202, no. 2, 15 July 2010 (2010-07-15) , pages 234-241, XP055206285, ISSN: 0022-1899, DOI: 10.1086/653621
- G. B. TRIANA-BALTZER ET AL: "DAS181, a sialidase fusion protein, protects human airway epithelium against influenza virus infection: an in vitro pharmacodynamic analysis", JOURNAL OF ANTIMICROBIAL CHEMOTHERAPY, vol. 65, no. 2, 26 November 2009 (2009-11-26), pages 275-284, XP055206288, ISSN: 0305-7453, DOI: 10.1093/jac/dkp421
- BROEDERS, ME ET AL.: 'Inhalation Profiles In Asthmatics And COPD Patients: Reproducibility And Effect Of Instruction.' J AEROSOL MED. SUMMER vol. 16, no. 2, 2003, pages 131 - 141, XP055080980
- CHIEN, MD, MS, JW ET AL.: 'Chapter 22: Influenza And Other Viral Respiratory Tract Infections.' EXPERT GUIDE TO INFECTIOUS DISEASES. 2008, page 424, XP055161994
- MATTHEWS ABIGAIL A. ET AL: "Developing inhaled protein therapeutics for lung diseases", MOLECULAR BIOMEDICINE, vol. 1, no. 1, 1 December 2020 (2020-12-01), XP055893710, DOI: 10.1186/s43556-020-00014-z Retrieved from the Internet: URL:https://link.springer.com/content/pdf/ 10.1186/s43556-020-00014-z.pdf>

## Description

### BACKGROUND

Human parainfluenza viruses (PIVs) are common causes of respiratory tract disease. The clinical and epidemiologic features of the four human PIVs differ. PIV-1 and PIV-2 infection are associated with laryngotracheobronchitis or swelling around the vocal chords and other parts of the upper and middle airway. PIV-3 is often associated with bronchiolitis and pneumonia. PIV-4 generally causes milder symtoms than the other types of human PIV.

Influenza viruses (IFV) can cause infections that affect mainly the nose, throat, bronchi and lungs. Infection is characterized by sudden onset of high fever, aching muscles, headache and severe malaise, non-productive cough, sore throat and rhinitis. Some influenza viruses are transmitted easily from person to person via droplets and small particles produced when infected people cough or sneeze. Most infected people recover within one to two weeks without requiring medical treatment. However, in the very young, the elderly, and those with other serious medical conditions, infection can lead to severe complications of the underlying condition, pneumonia and death. Moreover, certain strains and types of influenza viruses can cause serious illness even in healthy adults.

Dry powder inhalers are commonly used to administer drugs to the airway, e.g., the lungs. However, for some patients, e.g, children, particularly those under age 5, the elderly, immunocomprimised patients, and the severly ill, dry powder inhalers can be difficult to use effectively.

Moscona *et al.,* 2010 describes a recombinant sialidase fusion protein inhibiting human parainfluenza viral infection *in vitro* and *in vivo.* Triana-Baltzer *et al.,* describes a review of DAS181, a sialidase fusion protein protecting human airway epithelium against influenza infection. US 2007/190163 describes technology for preparation of macromolecular microspheres. US 2011/171132 describes methods, compounds and compositions for treatment and prophylaxis in the respiratory tract.

### FIGURES

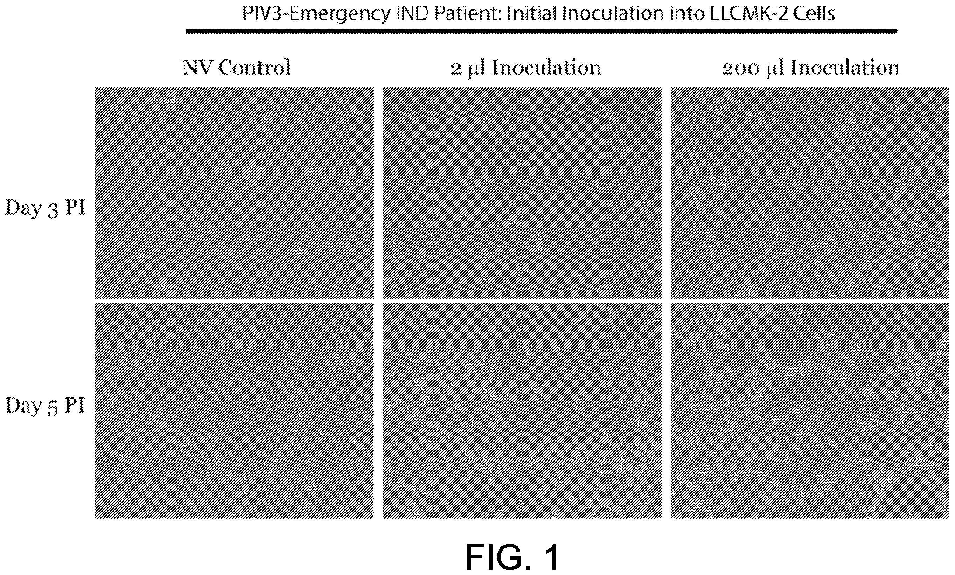

Figure 1 is a set of photographs depicting viral growth and culture. LLCMK-2 cells were seeded 24 hours prior to infection, and then were inoculated with either 0.02 or 0.2 mL viral culture
   positive specimen. Cells were just sub-confluent prior to infection. Infection progress was recorded 3 and 5 days post inoculation. Cellular death and CPE is detectable by 3 days post-inoculation, and has progressed significantly by 5 days post inoculation. Both 0.02 and 0.2 mL initial inoculum are sufficient to initiate infection. NV=Non-Viral. PI=Post Infection.
Figure 2 is set of photographs depicting the results of DFA analysis. Cells were grown on coverslips, infected at the TFID50, and then fixed 72 hours post-infection. Following fixation, cells were stained using the Light Diagnostics PIV3 DFA assay. Fluorescence was visualized under the microscope using channels specific for staining, and pictures were taken using ProgRes CapturePro software (Jenoptik).

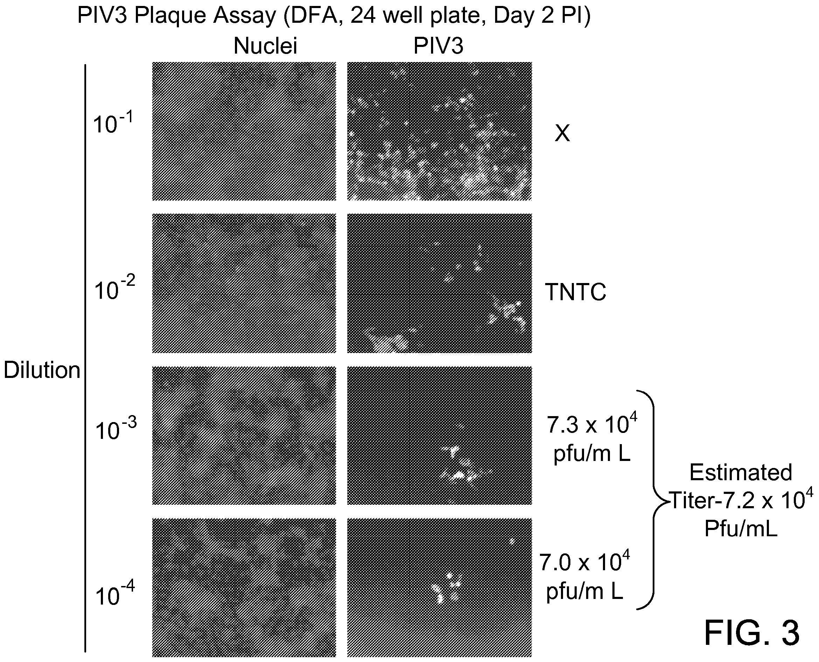

Figure 3 is set of photographs depicting representative plaques using DFA reagent.
   Plaque assay was conducted in a 24 well plate, and then stained using the DFA reagent as described in the Materials and Methods. Representative plaques from each dilution are shown, and the final titer obtained from counting all plaques in the wells is shown on the right side of the images. To obtain titer, the countable wells were averaged and multiplied by the dilution. Plaques are represented in green, while nuclei are represented in blue. TNTC=Too numerous to count. PI=post infection.

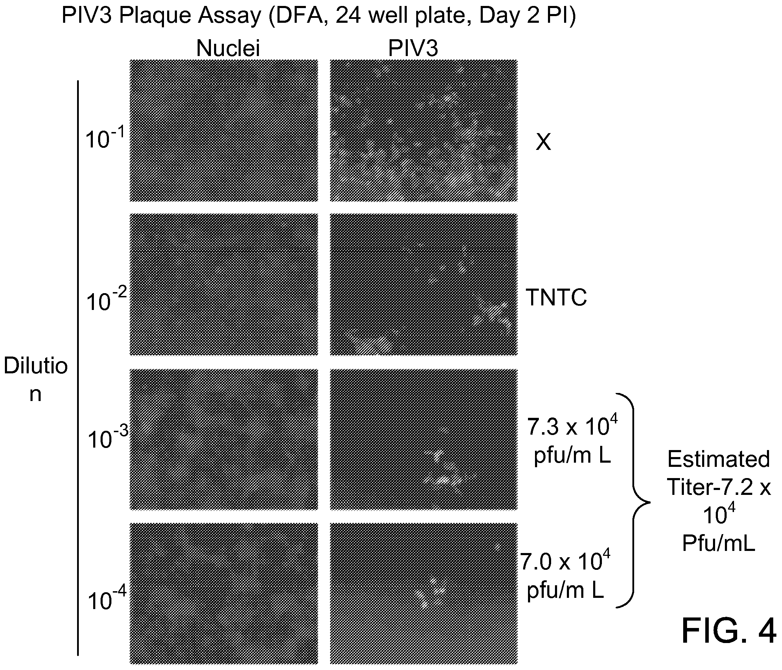

Figure 4 is set of photographs depicting representative plaques using DFA reagent.
   Plaque assay was conducted in a 24 well plate, and then stained using the DFA reagent as described in the Materials and Methods. Representative plaques from each dilution are shown, and the final titer obtained from counting all plaques in the wells is shown on the right side of the images. To obtain titer, the countable wells were averaged and multiplied by the dilution. Plaques are represented in green, while nuclei are represented in blue. TNTC=Too numerous to count. PI=post infection.

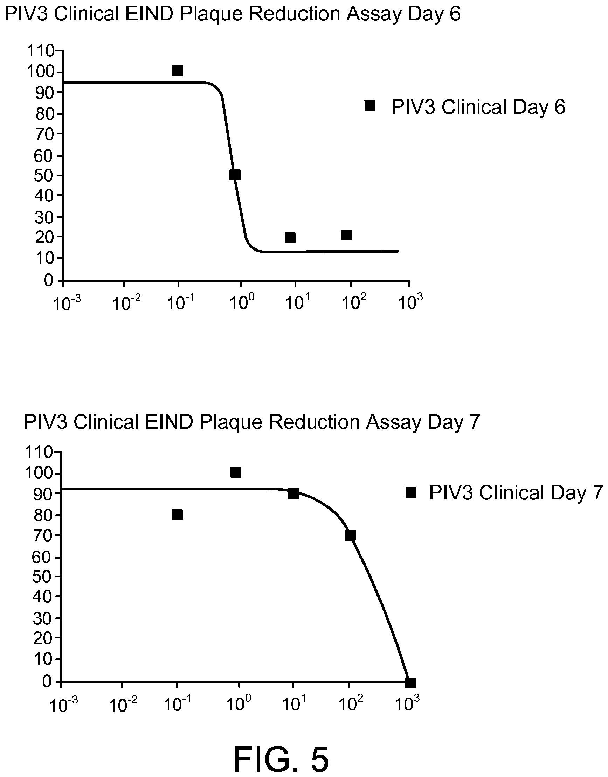

Figure 5 is a pari of graphs depicting starndard plaque reduction assay on day 6 and day 7. Plaque reduction assays were conducted in 6 well lates with increasing concentrations of DAS181, and were fixed either 6 or 7 days post-infection when plaques could be visualized. DAS181 remained in the overlay throughout the assay. Plaques were counted and graphed to determine EC50 values.

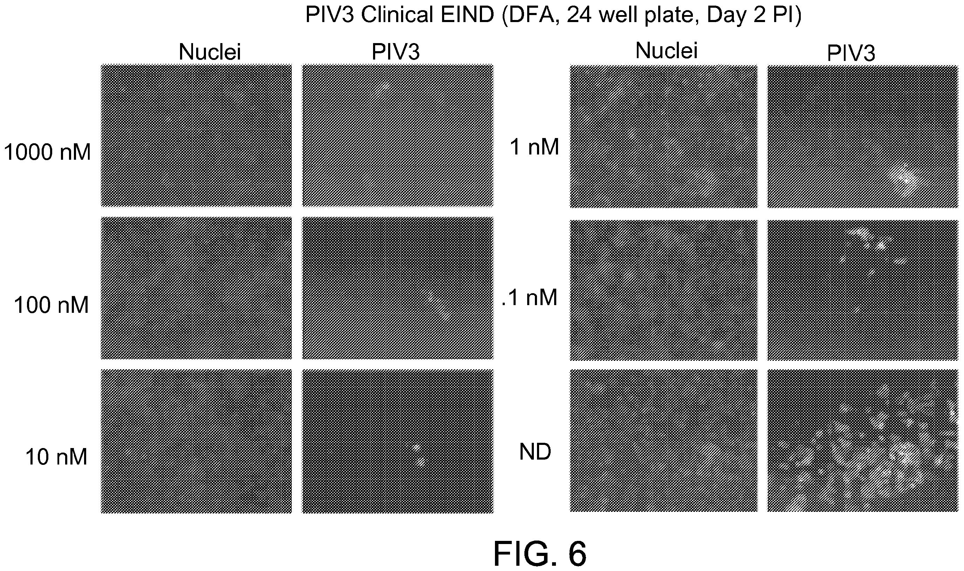

Figure 6 is a set of photographs depicting representative plaque formation using fluorescence analysis. Forty eight hours post-infection, cell were fixed and plaque formation was visualized using the PIV3 DFA reagent. Plaques are represented in gree, while nuceltic are represented in blue.

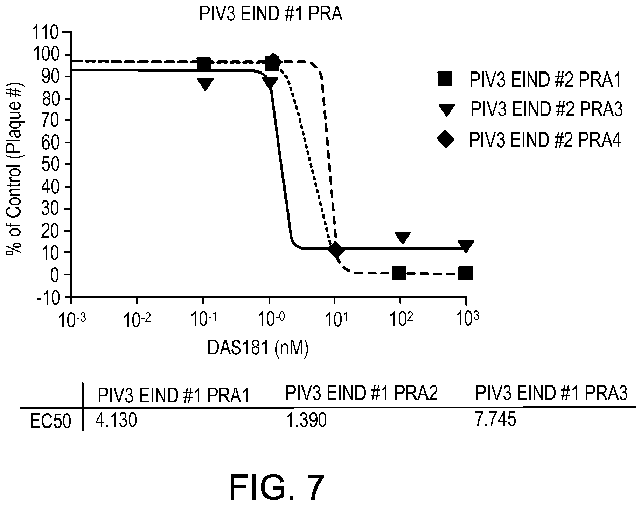

Figure 7 is a graph depicting plaque reduction assays conducted in triplicate and EC50 values were determined for each assay. An average EC50 vlaue of about 4 nM was established using these values.

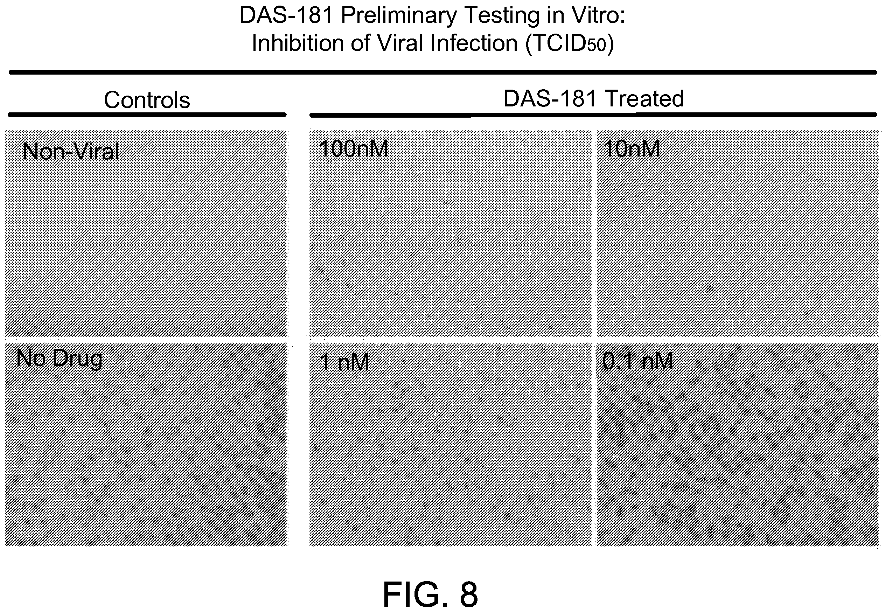

Figure 8 is a set of photographs depicting inhibition of TCID50. LLCMK-2 cell were seeded 24 hrs prior to infection, and were then infected with the knownTCID50. Two hours post infection, plates were fixed with 0.05% glutaldehyde, and then stained with an alphaPIV antibody. Dark starin represents the spread of virus.

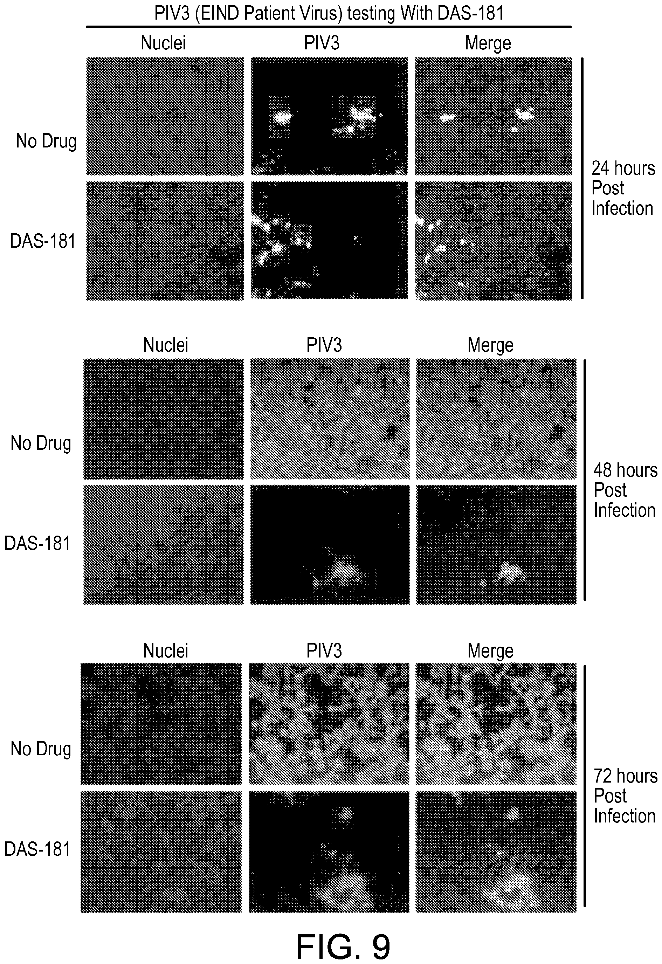

Figure 9 is a set of photographs depicting viral spread analysis. Cells were infected at a MOI of 0.1 and then assayed for viral spreatd 24, 48 and 72 hrs post infection with or without DAS 181 treatment (10 nM). Cells were fixed and then stained with PIV3 specific DFA reagent and visualized under the microscope. The presense of PIV3 infection is represented in green, while nucleit are represented in blue.

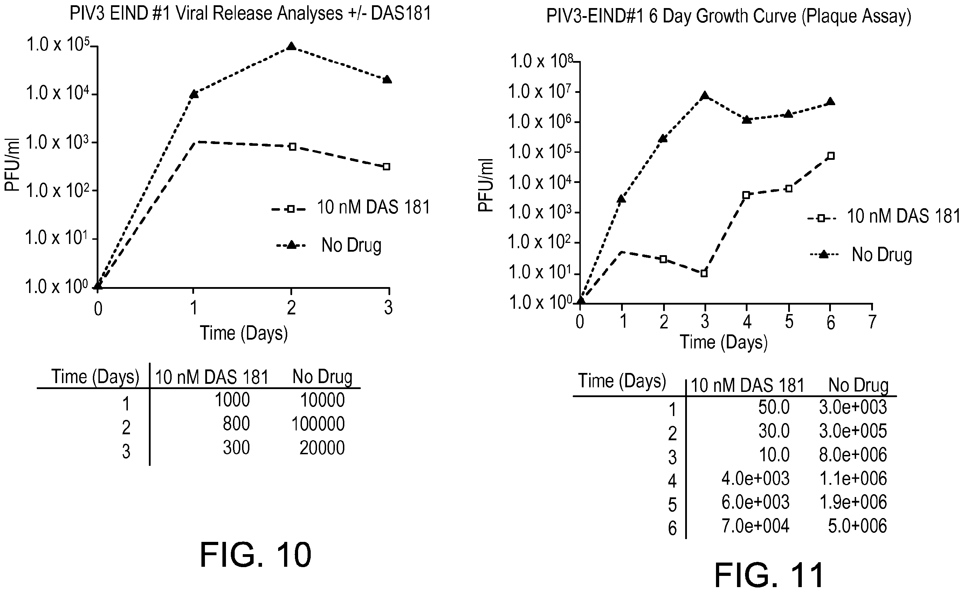

Figure 10 is a graph depicting viral release with 10 nM DAS 181 treatement. Following infection (MOI = 0.1) cells were treated (or mock treated) with 10 nM DAS181, and then infectious virus released from the cells ws measured by plaque assay. Tissue culture supernatants (with and withour DAS 181) were tested on Day 1, 2 and 3, and the tier was plotted over time.
Figure 11 is a graph depicting 6 Day PIV3 viral release study. Cells were infected at a low MOI (0.01) and then treated or mock treated with 10 nM DAS181. Viral release was measured by collection of tissue culture supernatant and infectious virus was assayed by plaque assay.

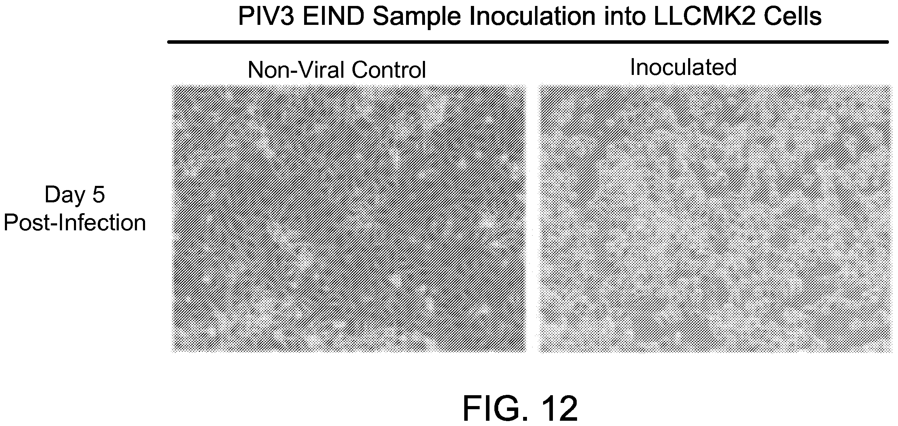

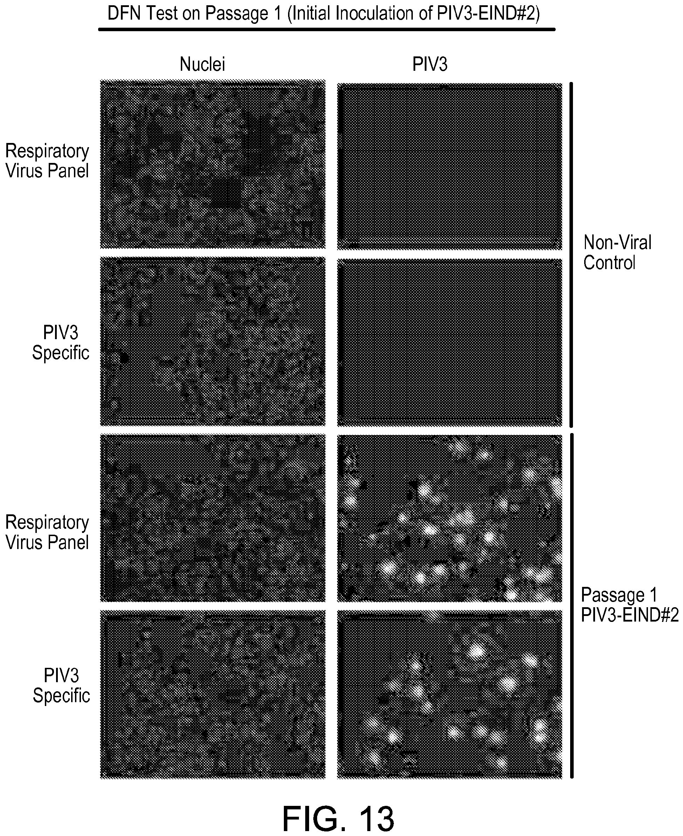

Figure 12 is a set of photographs depicting initial inoculation of patient sample. Samples suspected to contain PIV3 collected from an EIND patient were used to inoculate LLCMK2 cells. Cultures were monitored for CPE and evidence of viral infection. By Day 5 post-infection, the wells inoculated with 0.2 mL of the patient sample exhibited substantial CPE and cellular death, indicative of viral infection. Virus for further amplification was collected from these wells. The presence of PIV3 was confirmed by DFA assay (Figure 13). The NV control shows no positive staining for PIV3 antigen, while the infected PIV3 sample shows that all cells
   in the field are positive for PIV3 (represented in green). The nuclei are represented in blue.
Figure 13 is set of photographs depicting identification of viral type. Inoculated viral cultures were tested for the presence of a respiratory virus, as well as for the presence of PIV3 specifically by DFA analyses. Infected cells were spotted onto a glass slide and stained with appropriate antibodies (either recognizing a panel of respiratory viruses or specific for PIV3). Fluorescence was visualized under the microscope using channels specific for the staining, and pictures were taken using ProgRes CapturePro software (Jenoptik).

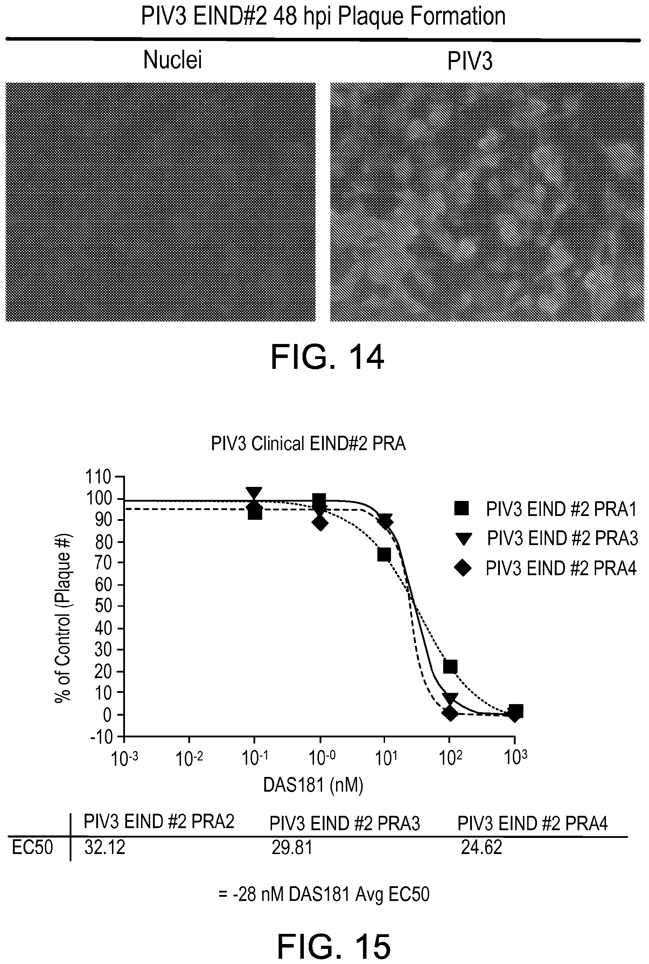

Figure 14 is set of photographs depicting representative plaque using DFA reagent.
   Plaque assay was conducted in a 24 well plate, and then stained using the DFA reagent as described in the
   Materials and Methods. A representative plaque from one dilution is shown, demonstrating the rapid spread of the viral plaque by 48 hours. To obtain titer, the countable wells were averaged and multiplied by the dilution. Plaque is represented in green, while all cells in the field are represented in blue.
Figure 15 is a graph depicting plaque reduction assay (PRA). Plaque reduction assays were conducted in triplicate, and EC50 values were determined for each assay. An average EC50 value of ~28 nM was established using these values.

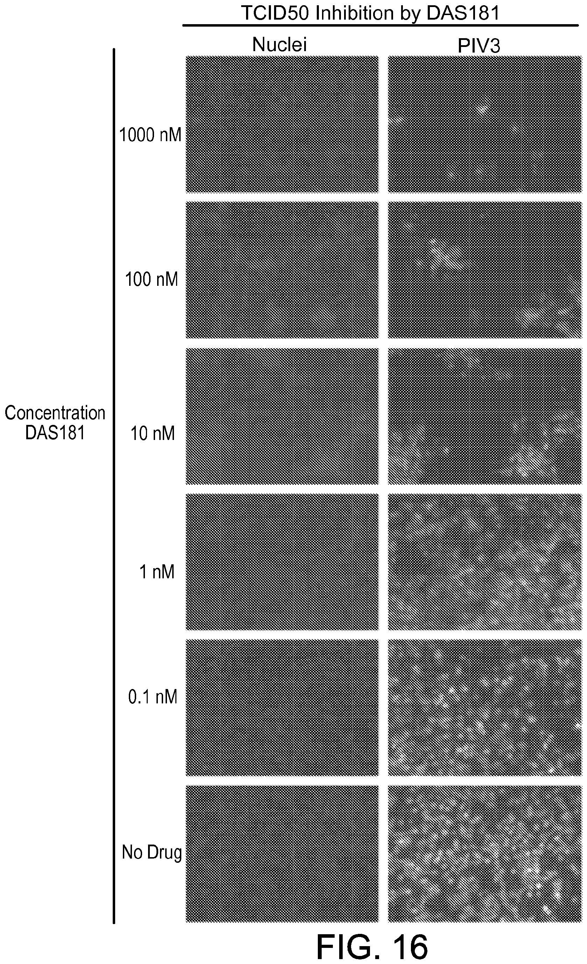

Figure 16 is a set of photographs depicting inhibition of TCID50. LLCMK-2 cells were seeded 24 hour prior to infection, and then were infected with the known TCID50. 2 hours post infection, plates were washed to remove residual virus, and were then overlayed with agarose/media containing serially diluted DAS 181. 3 days post infection, plates were fixed with 0.05% glutaraldehyde, and then stained with an aPIV3 antibody. Green fluorescence indicates the spread of the virus throughout the monolayer, whereas all cells are indicated by the blue stain.

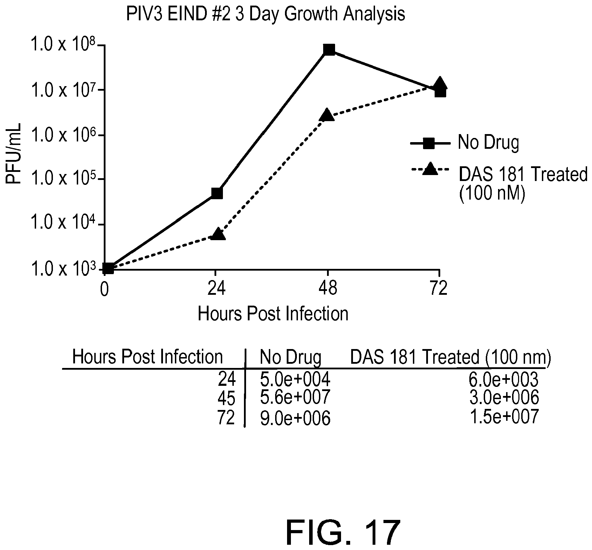

Figure 17 is a graph depicting the results of a 3 day PIV3 viral release study. Cells were infected at a low MOI (0.01) and then treated ormock treated with 100 nM DAS 181. Viral release was measured by collection of tissue culture supernatant and infectious virus was assessed by plaque assay.

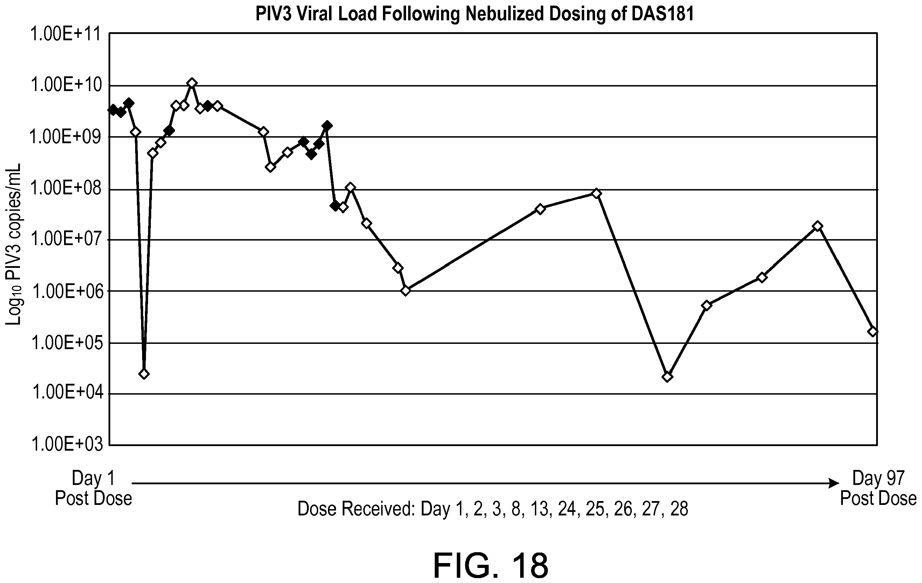

Figure 18 is a graph depicting the reduction in viral load (dosing days are indicated in red).

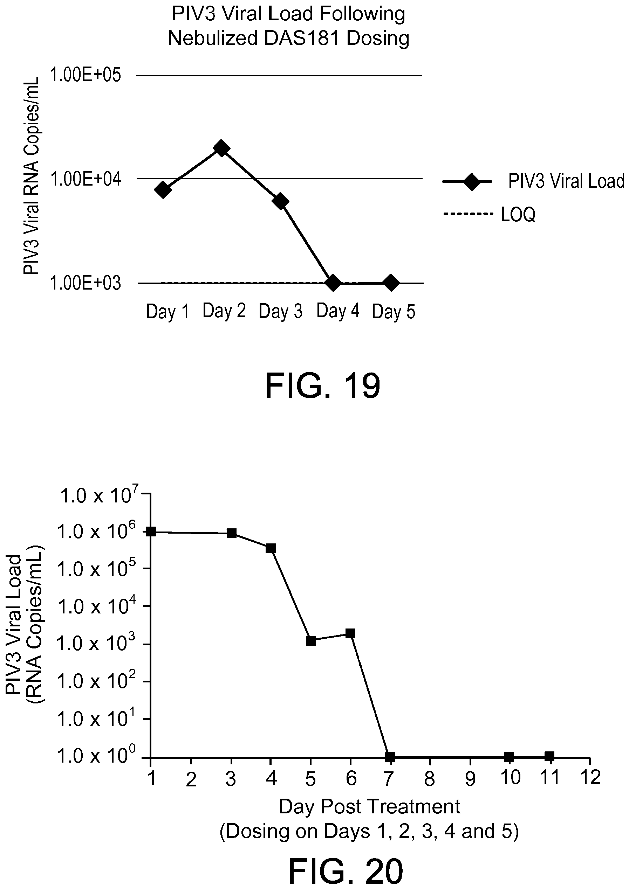

Figure 19 is graph depicting changes in viral load.
Figure 20 is a graph depicting changes in viral load as a function of day of treatment.

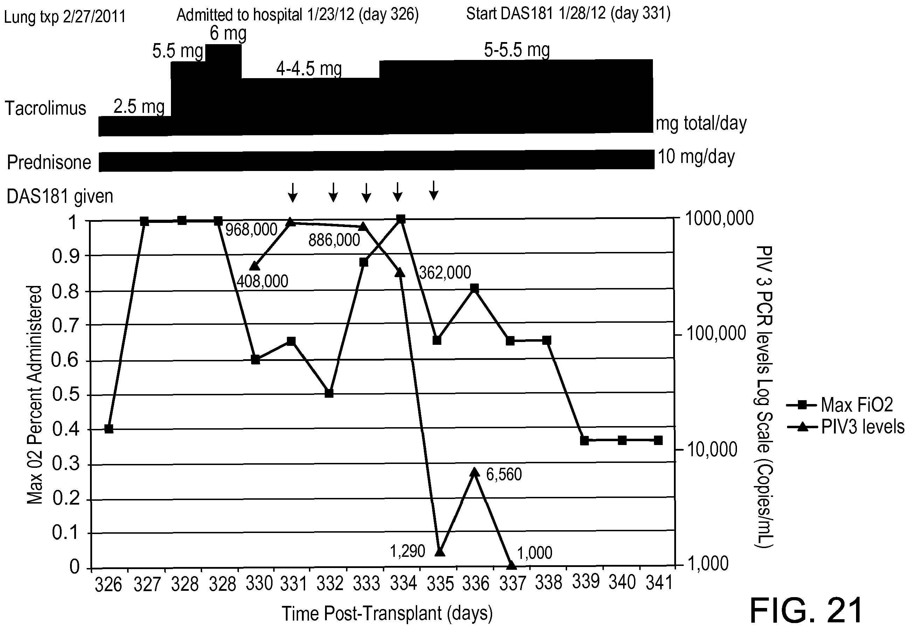

Figure 21 is a set of graphs depicting prednisone and tacrolimus treatment, oxygen administration and PIV3 load.

### SUMMARY

Aspects of the invention for which protection is sought are defined in the appended set of claims. The description may contain additional technical information, which although not part of the claimed invention, is provided to place the invention in a broader technical context and to illustrate possible related technical developments. References herein to methods of treatment of the human or animal body are to be understood as references to medicaments for use in a method of treatment.

In one aspect, the invention provides a composition comprising a therapeutically effective amount of a protein comprising SEQ ID NO: 1 or SEQ ID NO: 2 for use in a method of treating parainfluenza or influenza virus infection, the method comprising administering said composition to the respiratory tract of a patient, wherein the composition is in liquid form and the administration is by nebulizer, wherein 0.1 - 10mg of the protein is administered to the patient per day.

Described herein are methods and formulations for treating patients using liquid (e.g, nebulized) formulations of proteins, e.g., fusion proteins, having sialidase activity (e.g., DAS 181). The methods and formulations can be used to treat patients infected with PIV or influenza virus (IFV). Also described herein are methods for treating PIV infection in immunocompromised patients using proteins, e.g., fusion proteins, having sialidase activity (e.g., DAS181). Such immunocompromised can be treated with dry formulations or liquid (e.g., nebulized) formulations.

Useful proteins having sialdiase activity include DAS181, a 46-kDa recombinant fusion protein consisting of a sialidase functional domain fused with an amphiregulin glycosaminoglycan-binding sequence that anchors the sialidase to the respiratory epithelium. By cleaving sialic acids (SAs) from the host cell surface, DAS 181 inactivates the host cell receptors recognized by both PIV and IFV and thereby potentially renders the host cells resistant to PIV and IFV infection.

Described herein is a method for treating PIV or IFV infection in a patient, the method comprising: administering to the respiratory tract of the patient a compositon comprising a therapeutically effective amount of a liquid composition (e.g., a nebulized composition) comprising a protein having sialidase activity. Also described herein is a method for treating a subject at risk for PIV or IFV infection, the method comprising: administering to the respiratory tract of the subject a compositon (e.g., a therapeutically effective amount of a composition) comprising a liquid composition (e.g., a nebulized composition) or a dry powder formulation comprising a protein having sialidase activity. In various cases: the patient is an immunocompromised patient; the patient is suffering from a primary immunodeficiency; the immunocompromised patient is suffering from a secondary immunodeficiency; the immunocompromised patient is being or has been treated with an immunosuppressive therapy; the immunocompromised patient is being or has been treated with a chemotherapeutic agent; the immunocompromised patient is a transplant patient; the protein comprises or consistis of an amino acid sequence that is at least 90% (95%, 98%) identical or completely identical to SEQ ID NO: 1 or SEQ ID NO:2; the protein is DAS181; the composition further comprises one or more additional compounds; the administration is by use of a dry powder inhaler; the administration is by use of a nasal spray; the administration is by use of a nebulizer; the administration is by use of an endotracheal tube (ET tube), and a dry powder inhaler; the protein comprises a sialidase or an active portion thereof. In some cases: the sialidase or active portion thereof comprises an amino acid sequence that is at least 90%, 95%, 98%, 99% or 100% identical to: *Actinomyces viscosus* sialidase or its catalytic domain, *Clostridium perfringens* sialidase or its catalytic domain, *Arthrobacter ureafaciens* sialidase or its catalytic domain, *Micromonospora viridifaciens* sialidase or its catalytic domain, human Neu2 sialidase or its catalytic domain, or human Neu4 sialidase or its catalytic domain; and in other cases, the sialidase or active portion thereof is at least 90% identical to *Actinomyces viscosus* sialidase or its catalytic domain. In some cases: the peptide comprises an anchoring domain, wherein the anchoring domain is a glycosaminoglycan (GAG) binding domain (e.g., the GAG-binding domain is at least 90%, 95%, 98%, 99% or 100% idneitical to the GAG-binding domain of human platelet factor 4, the GAG-binding domain of human interleukin 8, the GAG-binding domain of human antithrombin III, the GAG-binding domain of human apoprotein E, the GAG-binding domain of human angio-associated migratory protein, or the GAG-binding domain of human amphiregulin).

In some cases the patient has insufficient pulmonary function to make effective use of dry powder inhaler or unable to use dry powder inhaler at all, e.g. patients on mechanical ventilator. In some cases the patient is an immunocompromised patient infected with PIV and is treated with a liquid formulation (e.g., using a nebulizer) or is treated with a dry formulation (e.g., using a dry powder inhaler).

In some cases the immunocompromised patients can include patients with malignancies, leukemias, collagen-vascular diseases, congenital or acquired immunodeficiency, including AIDS, organ-transplant recipients receiving immunosuppressive therapy, and other patients receiving immunosuppressive therapy.

Other features and advantages of the invention will be apparent from the following detailed description and figures, and from the claims.

### DETAILED DESCRIPTION

Described below are studies showing that DAS181, a fusion protein having silidase activity is effective against clinical isolates of PIV and in PIV infected patients. Various proteins having sialidase activity are described in US Patent No. 8,084,036; and DAS181 is described in US Patent No. 7,807,174.

DAS 181 is a fusion protein comprising a catalytic domain of a sialidase, and an anchoring domain. In some cases isolated DAS181 has an amino terminal methionine (Met) and in some cases it does not. Herein, the term DAS181 refers to either form or a mixture of the two forms, the sequences of which are provided herein as **SEQ ID NO: 1** and **SEQ ID NO:2.** Several of the examples described herein use DAS 181 or compositions containing DAS181.

DAS 181 and other proteins having sialidase activity, for example proteins described in US Patent No. 8,084,036 or US Patent No. 7,807,174 can be included in pharmaceutical compositions that are delivered to respiratory tract in a liquid formulation or a dry formulation.

The proteins described herein can be formulated into pharmaceutical compositions that include various excipients. In some cases, the formulations can include additional active ingredients that provide additional beneficial effects.

The present invention includes methods that use therapeutic compounds and compositions that comprise at least one sialidase activity. Proteins that are at least 90%, 95%, 98%, or 99% identical to SEQ ID NO: 1 or SEQ ID NO:2 are among those that can be useful. In some cases the amino acids that differ from those in SEQ ID NO: 1 or SEQ ID NO:2 are conservative substitutions. Conservative substitutions may be defined as exchanges within one of the following five groups:
I. Small, aliphatic, nonpolar or slightly polar residues: Ala, Ser, Thr, Pro, Gly
II. Polar, negatively charged residues and their amides: Asp, Asn, Glu, Gln
III. Polar, positively charged residues: His, Arg, Lys
IV. Large, aliphatic nonpolar residues: Met, Leu, Ile, Val, Cys
V. Large aromatic residues: Phe, Try, Trp

Within the foregoing groups, the following substitutions are considered to be "highly conservative": Asp/Glu, His/Arg/Lys, Phe/Tyr/Trp, and Met/Leu/Ile/Val. Semi-conservative substitutions are defined to be exchanges between two of groups (I)-(IV) above which are limited to supergroup (A), comprising (I), (II), and (III) above, or to supergroup (B), comprising (IV) and (V) above. In addition, where hydrophobic amino acids are specified in the application, they refer to the amino acids Ala, Gly, Pro, Met, Leu, Ile, Val, Cys, Phe, and Trp, whereas hydrophilic amino acids refer to Ser, Thr, Asp, Asn, Glu, Gln, His, Arg, Lys, and Tyr.

Dosage forms or administration by nebulizers generally contain large amounts of water in addition to the active ingredient. Minor amounts of other ingredients such as pH adjusters, emulsifiers or dispersing agents, preservatives, surfactants, or buffering and other stabilizing and solubilizing agents can also be present.

Nasal formulations can be administered as drops, sprays, aerosols or by any other intranasal dosage form. Optionally, the delivery system can be a unit dose delivery system. The volume of solution or suspension delivered per dose can be anywhere from about 5 to about 2000 microliters, from about 10 to about 1000 microliters, or from about 50 to about 500 microliters. Delivery systems for these various dosage forms can be dropper bottles, plastic squeeze units, atomizers, nebulizers or pharmaceutical aerosols in either unit dose or multiple dose packages.

The liquid formulations of this invention can be varied to include; (1) other acids and bases to adjust the pH; (2) other tonicity imparting agents such as sorbitol, glycerin and dextrose; (3) other antimicrobial preservatives such as other parahydroxy benzoic acid esters, sorbate, benzoate, propionate, chlorbutanol, phenylethyl alcohol, benzalkonium chloride, and mercurials; (4) other viscosity imparting agents such as sodium carboxymethylcellulose, microcrystalline cellulose, polyvinylpyrrolidone, polyvinyl alcohol and other gums; (5) suitable absorption enhancers; (6) stabilizing agents such as antioxidants, like bisulfite and ascorbate, metal chelating agents such as sodium edetate and drug solubility enhancers such as polyethylene glycols; and (7) other agents such as amino acids.

The invention includes liquid pharmaceutical compositions that at various dosage levels, such as dosage levels of DAS181 (or another polypeptide having sialidase activity) between about 0.1 and about 10 mg. Examples of such dosage levels include doses of about 0.1 mg, 0.5 mg, 1 mg, 5 mg or 10 mg/day. The foregoing doses can be administered one time per day, for one day, two days, three days, four days, five days, six days, seven days, eight days, nine days, ten days, eleven days, twelve days, thirteen days, or fourteen or more days. In various examples described herein, mice were treated with various dosages of the compositions described herein, including dosages of 0.0008 mg/kg, 0.004 mg/kg, 0.02 mg/kg, 0.06 mg/kg, 0.1 mg/kg, 0.3 mg/kg, 0.6 mg/kg, 1.0 gm/kg, 2.0 mg/kg, 3.0 mg/kg, 4.0 mg/kg and 5.0 mg/kg.

A "sialidase" is an enzyme that can remove a sialic acid residue from a substrate molecule. The sialidases (N-acylneuraminosylglycohydrolases, EC 3.2.1.18) are a group of enzymes that hydrolytically remove sialic acid residues from sialo-glycoconjugates. Sialic acids are alpha-keto acids with 9-carbon backbones that are usually found at the outermost positions of the oligosaccharide chains that are attached to glycoproteins and glycolipids. One of the major types of sialic acids is N-acetylneuraminic acid (Neu5Ac), which is the biosynthetic precursor for most of the other types. The substrate molecule can be, as nonlimiting examples, an oligosaccharide, a polysaccharide, a glycoprotein, a ganglioside, or a synthetic molecule. For example, a sialidase can cleave bonds having alpha(2,3)-Gal, alpha(2,6)-Gal, or alpha(2,8)-Gal linkages between a sialic acid residue and the remainder of a substrate molecule. A sialidase can also cleave any or all of the linkages between the sialic acid residue and the remainder of the substrate molecule. Two major linkages between Neu5Ac and the penultimate galactose residues of carbohydrate side chains are found in nature, Neu5Ac alpha (2,3)-Gal and NeuSAc alpha (2,6)-Gal. Both NeuSAc alpha (2,3)-Gal and NeuSAc alpha (2,6)-Gal molecules can be recognized by influenza viruses as the receptor, although human viruses seem to prefer NeuSAc alpha (2,6)-Gal, avian and equine viruses predominantly recognize Neu5Ac alpha (2,3)-Gal. A sialidase can be a naturally-occurring sialidase, an engineered sialidase (such as, but not limited to a sialidase whose amino acid sequence is based on the sequence of a naturally-occurring sialidase, including a sequence that is substantially homologous to the sequence of a naturally-occurring sialidase). As used herein, "sialidase" can also mean the active portion of a naturally-occurring sialidase, or a peptide or protein that comprises sequences based on the active portion of a naturally-occurring sialidase.

A "fusion protein" is a protein comprising amino acid sequences from at least two different sources. A fusion protein can comprise amino acid sequence that is derived from a naturally occurring protein or is substantially homologous to all or a portion of a naturally occurring protein, and in addition can comprise from one to a very large number of amino acids that are derived from or substantially homologous to all or a portion of a different naturally occurring protein. In the alternative, a fusion protein can comprise amino acid sequence that is derived from a naturally occurring protein or is substantially homologous to all or a portion of a naturally occurring protein, and in addition can comprise from one to a very large number of amino acids that are synthetic sequences.

A "sialidase catalytic domain protein" is a protein that comprises the catalytic domain of a sialidase, or an amino acid sequence that is substantially homologous to the catalytic domain of a sialidase, but does not comprise the entire amino acid sequence of the sialidase the catalytic domain is derived from, wherein the sialidase catalytic domain protein retains substantially the same activity as the intact sialidase the catalytic domain is derived from. A sialidase catalytic domain protein can comprise amino acid sequences that are not derived from a sialidase, but this is not required. A sialidase catalytic domain protein can comprise amino acid sequences that are derived from or substantially homologous to amino acid sequences of one or more other known proteins, or can comprise one or more amino acids that are not derived from or substantially homologous to amino acid sequences of other known proteins.

"Therapeutically effective amount" means an amount of a composition or compound that is needed for a desired therapeutic, prophylactic, or other biological effect or response when a composition or compound is administered to a subject in a single dosage form. The particular amount of the composition or compound will vary widely according to conditions such as the nature of the composition or compound, the nature of the condition being treated, the age and size of the subject.

"Treatment" means any manner in which one or more of the symptoms of a condition, disorder or disease are ameliorated or otherwise beneficially altered. Treatment also encompasses any pharmaceutical use of the composition or compound herein, such as for reducing mucus in the respiratory tract.

"Respiratory tract" means the air passages from the nose to the pulmonary alveoli, including the nose, throat, pharynx, larynx, trachea, and bronchi, and it also includes the lungs, and is sometimes referred to by medical practitioners as the respiratory system.

"Inhaler" means a device for giving medicines in the form of a spray or dry powder that is inhaled (breathed in either naturally or mechanically forced in to the lungs) through the nose or mouth, and includes without limitation, a passive or active ventilator (mechanical with or without an endotracheal tube), nebulizer, dry powder inhaler, metered dose inhaler, and pressurized metered dose inhaler.

"Inhalant" is any substance that is inhaled through the nose or mouth.

"Excipient" as used herein means one or more inactive substances or compounds that either alone or in combination are used as a carrier for the active ingredients of a medication. As used herein "excipient" can also mean one or more substances or compounds that are included in a pharmaceutical composition to improve its beneficial effects or that have a synergistic effect with the active ingredient.

### EXAMPLES

### Example 1 - Clinical Isolate

Described below are *in vitro* studies demonstrating that DAS 181 can inhibit a clinical isolate of PIV. The studies are significant because clinical isolates of PIV more closely resemble PIV that infects patients than do laboratory strains of PIV. The effective concentration required to inhibit viral replication by 50% (EC50) established for this virus was ~4 nM DAS 181.

Viral growth analyses also demonstrated that without DAS181, whether infected at an MOI of 0.01 or 0.1, the virus progresses rapidly through the cell culture monolayer. In both cases, by day 3 post infection, significant cytopathic effect (CPE) and cell death was observed without treatment with DAS181. However, in the presence of 10 nM DAS181, the cellular layer remained in tact throughout the course of infection, and viral release as measured by plaque assay was substantially reduced. Together, these data indicate that DAS 181 is effective against this clinical isolate of PIV3, and is protective against virally induced cytotoxicity and cellular death.

### Study Design and Results

Specimens received on dry ice were store at -80°C until analysis. When ready for analysis, the samples were tested for virus using LLC-MK2 cells and assessed for viral infection (viral type and strain). When infection was confirmed, the virus was passaged 2 times, until amplification for viral stock was sufficient. Characterization of the growth properties of the virus and effective inhibitory doses of DAS 181 were established.

TESTING SCHEME
Obtain Clinical Isolates
Culture Virus on LLC-MK2 Cells
Test using DFA Test (PIV3)
No Viable Viral Isolate
Positive DFA Test
Stop
Screen for Individual Viral Type
PIV Strain Confirmed
Minimal Passage to Obtain
Stock of Isolate
Characterize DAS-181 Sensitivity

Specimens (BAL and Tissue Culture Positive Supernatant) were used for inoculation onto LLCMK2 cells following a brief low speed centrifugation to remove cells and obtain only supernatant. Direct fluorescence analyses (DFA) were performed for initial identification of any viral species using a respiratory virus DFA screen. The separated viral supernatant (0.02 or 0.2 mL) was inoculated onto a 6 well plate with appropriate labeling and identification procedures.

Supernatant from the wells containing the initial viral inoculum was placed into multiple wells of fresh cells containing viral growth medium (VGM). Cells were monitored for CPE as described above. At 3 days post infection, one well of each isolate was collected for DFA analysis.

Initial viral inoculations of LLC-MK2 cells were monitored for CPE for multiple days (varied depending on viral strain and growth properties). Observations such as cell death, syncytia formation, cell rounding or enlargement, and overall changes in cellular growth were documented. Approximately 3-5 days post inoculation (or when cells exhibit CPE), cells were frozen at between -70 to -80 °C to allow virus release. After amplification of the virus into a larger growth vessel, the virus was frozen at between -70 to -80 °C for long-term storage.

Passaging of Viral Samples: The duplicate wells of the above initial isolation were used to continue the growth of the virus. Upon substantial cell lysis/death, the supernatant was transferred to new cells. Virus from each passage of the virus was also frozen at between -70 and -80°C to preserve the viral stock. To amplify, the virus is passaged with uninfected cells until a substantial volume of high titer virus can be obtained. To freeze the virus at, 1% DMSO is added and the virus is frozen in aliquots between -70 and -80°C.

Confirming Respiratory Viral Antigens: Initial DFA analysis was used to screen for the presence of a respiratory viral pathogen (including Adenovirus, Influenza A, Influenza B, Parainfluenza Type 1, Parainfluenza Type 2, Parainfluenza Type 3, and Respiratory Syncytial Virus). DFA analyses were performed according to manufacturer's instructions (Cat. # 3137, Millipore, Temecula, CA). Following positive result with the screening test to indicate the presence of respiratory viral antigen, the viral strain was confirmed using components of the above kit that are specific for individual viral strains and subtypes. For analysis of the viral strain, cells were spotted onto slides (or grown on glass coverslips) to allow for appropriate analysis, as per manufacturer's instructions.

Identification of Viral Isolate: Following passage of the virus as described above, confirmatory DFA analysis was conducted on the specimen that yielded productive infection to confirm the identified viral subtype. Continued confirmation was conducted throughout viral studies at varied periods of time allowing monitoring for changes in viral type.

Freezing and Organization of Viral Stocks: Once the viral strain was identified and confirmed, viral stocks were amplified from the original isolate, and frozen at -70°C in multiple aliquots to ensure low passage. SOPs, and plaque assay modifications were made as described below. Low passage virus was used for all subsequent analysis, in order to maintain characteristics (both phenotypic and genotypic) that are as close to the original isolate as possible.

Titering of Viral Stocks: Virus stocks were titered on LLC-MK2 cell monolayers and assayed between day 2-7 postinfection by fixing with 0.05% glutaraldehyde or 4% formaldehyde, and then incubation with PIV-subtype specific antibodies and DFA reagents. Following staining, the plaques were counted and titer was determined according to counts.

Inhibition of TCID50: LLC-MK2 cells were plated in a 6 well plate 1 day prior to infection at a density of 3×10⁶ cells/plate. The following day, cells were washed with 1X PBS one time, and then infected at the identified TCID50 for the viral stock. 2 hours post-infection, cells were overlayed with agarose containing varying concentration of DAS 181 ranging from 1000 nM to 0.1 nM (10X serial dilutions). A no drug control as well as a non-viral (NV) control was also assessed. 3-5 days post infection (when cells exhibited substantial cytopathic effect), cells were fixed and then stained with an antibody specific to PIV2/3. Following staining with the antibody, plates were washed 3X with 1X PBS+0.05% Tween-20. Plates were then stained with the TBP/BCIP substrate for 10-15 minutes, or until staining was visible. Representative pictures were taken, and observations were made regarding the spread of the virus, as well as the level of inhibition provided by the DAS181 treatment.

Plaque Reduction Assay: A modified plaque reduction assay (PRA) was conducted to determine the level of DAS181 sufficient to inhibit the infection 50% (EC50). Cells were seeded the day before infection at a density of 3×10⁶ cells/plate in a 24 well plate. The next day, cells were washed with 1X PBS, and then infected with ≤100 pfu/well for 2 hours. After the initial 2 hours, media was aspirated, and cells were again washed in 1X PBS. Plates were overlayed with agarose in 2X Eagle's minimum essential media (EMEM) (1: 1 ratio) containing appropriate concentration of DAS 181 (1000 nM to 0.1 nM). Each concentration of DAS 181 was assayed in duplicate wells, and resulting plaque counts were averaged from the 2 wells. Plaques were allowed to form for 2 days, at which point plates were fixed with 0.05% Glutaraldehyde or 4% Formaldehyde. Following fixation, plates were stained with the appropriate antibody or DFA reagent according to manufacturer's instructions.

Viral Growth Curve (+/- DAS181) Using Plaque Assay: Viral release over time +/- DAS181 was assessed by seeding cells in a 24 well plate (3 × 10⁶ cells/plate) the day before infection. The next day, cells were infected at a low multiplicity of infection (MOI) (between 0.01 and 0.1), and 2 hours post infection, media was removed and replenished with fresh media with or without DAS 181 at identified concentration required to inhibit the virus. Viral supernatant was harvested every 24 hours until ~80-90% cellular death was evident in the control treated wells, and then media containing DAS 181 was replenished. Supernatant was frozen at -80°C, and then viral titer for each sample was assessed by standard plaque assay for PIV. Spread of the virus was also assessed using this experimental set-up, except that cells were grown on glass coverslips, and then fixed and stained as described above for plaque reduction assay.

Viral Growth Curve Using Quantitative Real-Time RT-PCR: The assay set-up described above (section 8.3) was also attempted for viral quantitation by quantitative real-time reverse transcription (RT-PCR). Viral supernatant was harvested as above, and then RNA was prepared. Equal volume of viral supernatant was used as starting material, and a control RNA (GAPDH) was spiked into each sample to control for differences in the amount of RNA isolated from each sample due to purification differences between samples. RNA was then analyzed in a one-step RT-PCR reaction.

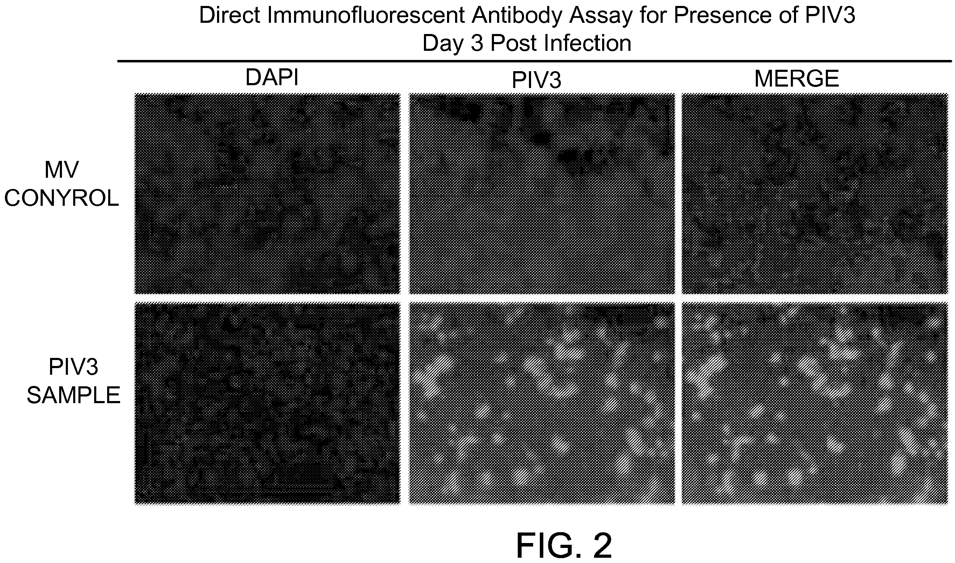

Initial Inoculation of PIV3 Samples: Cultures were inoculated with either 0.02 or 0.2 mL of patient sample (either a BAL or previously identified positive tissue culture supernatant). Cells were allowed to grow for 5 days, and were observed daily for CPE or other evidence of viral infection. At Day 3 and Day 5 post infection, pictures were taken and CPE was observed in cells inoculated with the tissue culture supernatant (**Figure 1**). The BAL samples did not yield productive viral infection, whereas the viral culture supernatants displayed signs of CPE as early as 3 days post infection. Cells inoculated with 0.2 mL viral supernatant displayed proportionally more CPE than the cells inoculated with 0.02 mL. By day 5 post infection, cells infected 0.2 mL viral supernatant had progressed substantially, and exhibited approximately 50% cell death, indicative of viral spread throughout the culture. The sample inoculated with 0.02 mL viral supernatant progressed further by this day, but was substantially less infected when compared to the sample inoculated with 0.2 mL. The BAL samples still exhibited no signs of viral infection by this time. Further observation (out to day 14) confirmed that no productive viral infection was isolated for this sample. The presence of PIV3 was confirmed by DFA assay (**Figure 2**). The NV control shows no positive staining for PIV3 antigen, while the infected PIV3 sample shows that all cells in the field are positive for PIV3

Plaque Assay to Determine Titer: PIV3 isolated from this patient was passaged minimally on LLC-MK2 cells, and then tittered using a modified plaque assay. Multiple variations of this standard assay were tested given that this virus did not plaque as readily and consistently as a PIV3 reference strain. Compared to previous PIV reference strain plaque assays, this virus took much longer to produce plaques that were visible to the eye when stained with the appropriate antibodies. By Day 6 post infection, plaques could be visualized although plaque size was variable and many were still much smaller in size. By Day 7, plaques were very easy to visualize, although variation in size was still noted (data not shown). In comparison, reference strains were easily and consistently visualized using this method by Day 3 post infection. In order to obtain accurate and consistent results with both the plaque assay and plaque reduction assay, both were modified to decrease the time in culture required for consistent plaque counts, as well as to increase the ability to visualize smaller plaques that are inherent in this particular viral isolate. The modified assay is based on the same principle as described for standard plaque assay/plaque reduction assay. However, because plaque formation of PIV does not require large surface area, the assay format was changed to be done in a 24 well plate set-up. Virus was serially diluted (10- 1-10-6) and duplicate wells were infected for plaque assay, and then virus was washed and overlayed in 2X MEM:Agarose mixture as described for normal plaque assay procedure. Infection was allowed to progress for 48 hours, and then cells were fixed and stained using the same DFA reagent used for identification and confirmation of viral type (**Figure 4**).

DAS181 Testing of Clinical PIV3 Isolate: In the standard plaque reduction assay, DAS 181 treatment was required for an extended period, up to 7 days, for visible plaques to develop. The amount of DAS181 required to inhibit the virus increased as the time remaining in culture increased as the pharmacological activity in the wells was lost. This time was deemed too long to achieve consistent, accurate inhibitory information (**Figure 5**). For the modified plaque reduction assay, virus was diluted to infect cells at 50 pfu/well in VGM. Two hours post infection, plates were washed and overlayed with 2X VGM:Agarose overlay containing serially diluted DAS181 (1000 nM-0.1 nM) or with no DAS181 for control. Viral infection was allowed to continue for 48 hours, and then cells were fixed and stained with the DFA reagent described above. Representative plaques for each dilution are shown in **Figure 6****.** Plaque size was also reduced as DAS 181 concentration increased. Graphs of the total counts per dilution are shown, and EC50 values for each graph are indicated (**Figure 7**). Plaque reduction assay was conducted 3 times on three different days to ensure accurate EC50 values across multiple days and passages. From these data, the average EC50 value established for this virus is ~4 nM.

DAS 181 Inhibition of TCID50 of the Clinical PIV3 Isolate: Given that viral production was greatly inhibited by DAS181 using the plaque reduction assay, we also tested the ability of the drug to inhibit virus at a higher multiplicity of infection. To accomplish this, cells were infected at the approximate TCID50 identified for the PIV3 clinical isolate, and then were treated with serially diluted DAS181 (0.1-100 nM) 2 hours post infection and overlayed with agarose. Five days post infection, cells were fixed and stained with an antibody specific for PIV3. Viral antigen was visualized using a secondary antibody conjugated with alkaline phosphatase, and then stained with a TBPBCIP substrate (**Figure 8**). Viral antigen is represented with the dark purple stain. Inhibition of viral infection was observed between 1-10 nM, whereas cells treated with 0.1 nM DAS 181 exhibited similar viral spread as the no drug control. In support of the plaque reduction assay, these results suggest that the inhibitory concentration of DAS181 required to limit spread of the viral infection was between 1-10 nM. Formation of pin-point plaques was observed in each of the wells that were infected; however the spread was substantially inhibited at all tested concentrations of DAS 181 above 0.1 nM.

DAS 181 Inhibition of Viral Spread and Release: To better quantify the inhibition of viral infection, viral growth analyses were conducted. First, viral spread was monitored throughout the course of infection (72 hours) using DFA analyses to monitor viral spread. To do this, cells were grown on glass coverslips and infected at an MOI of 0.1. Virus was removed 2 hours post infection, and cells were treated with DAS 181 (or mock treated with PBS) and then assayed for viral spread every 24 hours. Coverslips were stained with the PIV3 DFA reagent, and assessed for the presence of viral infection. At all times post infection, treatment with 10 nM DAS 181 significantly inhibited spread of the virus (**Figure 9**). The same cells as used in **Figure 9** were also assessed for viral release over time. Tissue culture supernatant was collected every 24 hours from the DAS181 treated or mock treated wells, and then frozen at -80°C until analysis. Viral titer (PFU/mL) was assessed by plaque assay as described above, and then graphed over time. The amount of virus released from DAS 181 treated cells was dramatically reduced over time (greater than 2 logs) when compared to untreated cells (**Figure 10**).

In order to assess viral release over a longer course of infection, cells were infected at a lower MOI (0.01), and then assessed as above. A similar trend was observed in that DAS181 treated cells produced significantly less infectious virus throughout the time course of infection (**Figure 11**). By Day 3 post infection, the viral infection in the untreated cells had progressed substantially, and all cells exhibited CPE. By Day 4 post infection, untreated cells began to die off (-50%), and by Day 6 the untreated wells exhibited ~90-95% cell death. In the DAS181 treated cells, viral CPE was not observed until Day 4 post infection, at which point a small percentage of cells exhibited signs of viral infection (rounding or cell death). By Day 6 post infection, the viral infection had spread slightly, but the majority of cells were still alive and exhibited minimal signs of CPE. Viral release also increased by Day 4 post infection in the DAS181 treated sample, although this was still considerably less than in the untreated sample.

### Conclusions

DAS 181 effectively inhibits this clinical strain of PIV3 at all concentrations between 1-10 nM. The established EC50 was ~4 nM, which is less than is required to inhibit most influenza strains that have been tested in this assay. DAS181 treatment over the course of infection in cell culture effectively reduces viral release over time by greater than 2 logs. Cytotoxicity and cell death induced by PIV3 infection is substantial by Day 3 post infection when infected at a low MOI. Modification of the standard plaque assay and plaque reduction assay allow increased consistency and feasibility of these assays. These data extend the current knowledge of the ability of DAS 181 to effectively inhibit different isolates of PIV, and demonstrates DAS181 inhibition of a PIV clinical strain.

### Materials and Methods

Cells and Viruses: Original LLC-MK2 cells were received from ATCC (Manassas, VA) and have been passaged minimal times (less than 4) to obtain multiple source vials. Cells were thawed prior to receipt of the subject specimens, and passaged at least 2 times before inoculation with the test sample.

Cell Culture Maintenance and Viral Growth Medium: Cells were split every 3-4 days, and fed every 2-3 days with Eagles-MEM (Cat. # 11095-098, Life Technologies, Carlsbad, CA), 10% FBS (Cat. # 14-502F, Lonza, Riverside, CA), 1X Glutamax (Cat. # 35050, GIBCO, Carlsbad, CA), and 1X Antibiotic/Antimycotic solution (Cat. # A5955, Sigma, St. Louis, MO). Cells were maintained in ample media according to standard protocols, and were grown at 37°C in a humidified chamber containing 5 % CO2, unless removed for maintenance or testing. Cells were washed with PBS (Cat. # 14040, GIBCO, Carlsbad, CA), and trypsinized using TrypLE Express (Cat# 12605-010, GIBCO, Carlsbad, CA). Individual flasks of cells were maintained according to standard protocols, and were labeled with the date of passage, initials of scientist, cell passage number, and the name of the cells. Viral infections were performed in the appropriate testing apparatus, including 6 or 24 well plates (Corning, Lowell, MA), as defined by the experiment. Cells were maintained in polystyrene flasks (Corning, Lowell, MA) during amplification before infection. Viral growth media consisted of E-MEM (listed above), 1X Glutamax (listed above), 3.0 mg/mL acetylated trypsin (Cat. # T6763, Sigma) diluted to a final concentration of 3.0 µg/mL, and 1% ITS (Cat. # 41400, GIBCO, Carlsbad, CA). Plaque assay overlay medium consisted of a 1:1 (vol:vol) mixture of the E-MEM media listed above (2X concentration) and 1.8% Noble Agar (Cat. # 10907, USB Corp., Cleveland, OH) in dH₂0 to achieve a final concentration of 1X media and 0.9% agarose. The lot of DAS181 used for these studies was Lot #20080715, prepared on 20-Jan-09 at a concentration of 25.5 mg/mL

RNA Extraction and Amplification: RNA extraction was performed using the QIAamp Viral RNA purification kit (Cat. # 52904, Qiagen, Valencia, CA) or the MagMAX^{™}-96 Total RNA Isolation Kit (Cat. # AM1830, Ambion, Foster City, CA) according to manufacturer's instructions. Amplification and quantitation of viral RNA was attempted using the TaqMan^{®} One-Step RT-PCR Master Mix Reagents Kit (Cat. # 4309169) according to the manufacturer's instructions. These analyses were initiated, although it was determined that the current established assay format was not reliable for these studies, and thus these data were not included in this report.

Antibodies and Staining Reagents: For TCID50 plate staining, αPIV2/3 (Cat. # 20-PG90, Fitzgerald, Acton, MA) antibody was used, followed by a donkey anti-goat secondary antibody (Cat. # V1151, Promega, Madison, WI). Antibody staining was visualized using the 1-Step NBT/BCIP substrate (Cat. # 34042, Thermo Scientific, Rockford, IL).

### Example 2 - PIV3 Clinical Isolate

### Study Design and Results

In a separate study a second clinical isolate of PIV3 was studied. In comparison to reference (laboratory adapted) strains, this clinical isolate grew faster in culture and formed plaques that readily spread through the culture within 24 hours. This virus also grew to very high titer, indicating this particular strain of PIV3 is highly virulent. The established EC50 for this virus is ~28 nM.

Viral growth analyses also demonstrated that without DAS181, when infected at an MOI of 0.01, the virus progresses rapidly through the cell culture monolayer. By day 3 post infection, significant CPE and cell death were observed without treatment with DAS181. However, in the presence of 100 nM DAS 181, the cellular layer remained predominantly intact throughout the course of infection, and viral release as measured by plaque assay was substantially reduced. Together, these data indicate DAS 181 is effective against this clinical isolate of PIV3, and is protective against virally induced cytotoxicity and cellular death.

Specimens were stored at -80°C until analysis. When ready for analysis, the samples were tested for virus using LLC-MK2 cells and assessed for viral infection (viral type and strain). When infection was confirmed, the virus was passaged 2 times, until amplification for viral stock was sufficient. Characterization of the growth properties of the virus and effective inhibitory doses of DAS 181 were established.

TESTING SCHEME
Obtain Clinical Isolates
Culture Virus on LLC-MK2 Cells
Test using DFA Reagent (PIV3)
No Viable Viral Isolate
Positive DFA Test
Stop
Screen for Individual Viral Type
PIV Strain Confirmed
Minimal Passage to Obtain
Stock of Isolate
Characterize DAS-181 Sensitivity

Inoculation of Clinical Specimens: Specimens (nasal swabs) were used for inoculation onto LLC-MK2 cells following a brief low speed centrifugation to remove cells and obtain only supernatant. Only the nasal swab collected before treatment with DAS181 allowed productive infection. DFAs were performed for initial identification of any viral species using a respiratory virus DFA screen. The separated viral supernatant (0.02 or 0.2 mL) was inoculated onto a 6 well plate with appropriate labeling and identification procedures. The presence of PIV3 antigen was tested with the DFA reagent specific for the viral type.

Isolation of Initial Inoculum: Supernatant from the wells containing the initial viral inoculum was placed into multiple wells of fresh cells containing viral growth medium (VGM). Cells were monitored for CPE as stated above. At 3 days post infection, one well of each isolate was collected for DFA .

Viral Amplification: Initial viral inoculations of LLC-MK2 cells were monitored for CPE for multiple days. Observations such as cell death, syncytia formation, cell rounding or enlargement, and overall changes in cellular growth were documented. Approximately 3-5 days post inoculation (or when cells exhibit CPE), cells were frozen at between -70 to -80 °C to allow virus release. After amplification of the virus into a larger growth vessel, the virus was aliquoted and frozen at between -70 to -80 °C for long-term storage.

Confirming Respiratory Viral Antigens: Initial DFA was used to screen for the presence of a respiratory viral pathogen (including Adenovirus, Influenza A, Influenza B, Parainfluenza Type 1, Parainfluenza Type 2, Parainfluenza Type 3, and Respiratory Syncytial Virus). DFA analyses were performed according to manufacturer's instructions (Cat. # 3137, Millipore, Temecula, CA). Following positive result with the screening test to indicate the presence of respiratory viral antigen, the viral strain was confirmed using components of the above kit that are specific for individual viral strains andsubtypes. For analysis of the viral strain, cells were spotted onto slides (or grown on glass coverslips) to allow for appropriate analysis, as per manufacturer's instructions.

Freezing and Organization of Viral Stocks: Once the viral strain was identified and confirmed, viral stocks were amplified from the original isolate, and frozen at at - 70°C in multiple aliquots to ensure low passage. Low passage virus was used for all subsequent analysis, in order to maintain characteristics (both phenotypic and genotypic) that are as close to the original isolate as possible.

Titering of Viral Stocks: Virus stocks were titered on LLC-MK2 cell monolayers and assayed on day 2 post-infection by fixing with 0.05% glutaraldehyde or 4% formaldehyde, and then incubation with PIV-subtype specific DFA reagent. Following staining, the plaques were counted and titer was determined according to counts.

Inhibition of TCID50: LLC-MK2 cells were plated in a 6 well plate 1 day prior to infection at a density of 3×10⁶ cells/plate. The following day, cells were washed with 1X PBS one time, and then infected at the identified TCID50 for the viral stock. 2 hours post infection, cells were overlayed with agarose containing varying concentrations of DAS 181 ranging from 1000 nM to 0.1 nM (10X serial dilutions). A no drug control as well as a non-viral (NV) control was also assessed. At 3-5 days post infection (when cells exhibited substantial CPE) cells were fixed and then stained with the PIV3 specific DFA reagent. Following staining with the antibody, plates were washed 3X with 1X PBS+0.05% Tween-20. Plates were then analyzed for viral spread. Representative pictures were taken, and observations were made regarding the spread of the virus, as well as the level of inhibition provided by the DAS181 treatment.

Plaque Reduction Assay: A modified plaque reduction assay (PRA) was conducted to determine the level of DAS181 sufficient to inhibit the infection 50% (EC50). Cells were seeded the day before infection at a density of 3×10⁶ cells/plate in a 24 well plate. The next day, cells were washed with 1X PBS, and then infected with ≤100 PFU/well for 2 hours. After the initial 2 hours, media was aspirated, and cells were again washed in 1X PBS. Plates were overlayed with agarose in 2X EMEM (1: 1 ratio) containing appropriate concentration of DAS181 (1000 nM to 0.1 nM). Each concentration of DAS 181 was assayed in duplicate wells, and resulting plaque counts were averaged from the 2 wells. Plaques were allowed to form for 2 days, at which point plates were fixed with 0.05% Glutaraldehyde or 4% Formaldehyde. Following fixation, plates were stained with the appropriate antibody or DFA reagent according to manufacturer's instructions.

Viral Growth Curve (+/- DAS181) Using Plaque Assay: Viral release over time +/- DAS181 was assessed by seeding cells in a 24 well plate (3×10⁶cells/plate) the day before infection. The next day, cells were infected at a multiplicity of infection (MOI) of 0.01, and 2 hours post infection, media was removed and replenished with fresh media with or without DAS181 at identified concentration required to inhibit the virus (100 nM). Viral supernatant was harvested every 24 hours until ~80-90% cellular death was evident in the control treated wells, and then media containing DAS181 was replenished. Supernatant was frozen at -80°C, and then viral titer for each sample was assessed by plaque assay.

Initial Inoculation of PIV3 Samples: Cultures were inoculated with either 0.02 or 0.2 mL of patient sample (either a BAL or previously identified positive tissue culture supernatant). Cells were allowed to grow for 5 days, and were observed daily for CPE or other evidence of viral infection. At Day 5 post infection, pictures were taken and CPE was observed in cells inoculated with 0.2 mL tissue culture supernatant (**Figure 12**). Cells inoculated with 0.2 mL viral supernatant displayed proportionally more CPE than the cells inoculated with 0.02 mL, and thus these wells were continued for viral propagation. By day 5 post-infection, cells infected 0.2 mL viral supernatant had progressed substantially, and exhibited approximately 50% cell death, indicative of viral spread throughout the culture. The sample inoculated with 0.02 mL viral supernatant progressed somewhat by this day, but was substantially less infected when compared to the sample inoculated with 0.2 mL.

Inoculated viral cultures were tested for the presence of a respiratory virus, as well as for the presence of PIV3 specifically by DFA. Infected cells were spotted onto a glass slide and stained with antibodies recognizing either a panel of respiratory viruses or specific for PIV3 (**Figure 13**).

Plaque Assay to Determine Titer: PIV3 isolated from this patient was passaged minimally on LLC-MK2 cells, and then tittered using a modified plaque assay. Compared to previous PIV reference strain plaque assays and to another clinical isolate of PIV3, this virus grew much faster and produced plaques that were visible to the eye when stained with the appropriate antibodies/staining reagent. By Day 2 post-infection, plaques could be visualized and many had spread significantly by this time. The modified assay used for this virus is based on the same principle as described for standard plaque assay/plaque reduction assay. However, because plaque formation of PIV does not require large surface area, the assay format was done in a 24 well plate set-up. Virus was serially diluted (10-1-10-6) and duplicate wells were infected for plaque assay, and then virus was washed and overlayed in 2X MEM:Agarose mixture as described for normal plaque assay procedure. Infection was allowed to progress for 36-48 hours, and then cells were fixed and stained using the same DFA reagent used for identification and confirmation of viral type (**Figure 14**). The identified titer of the infectious virus produced from this inoculation was 8×10⁶ PFU/mL. This is substantially higher (compared to 2×10⁵ PFU/mL) than the other clinical isolate of PIV3.

DAS181 Testing of Clinical PIV3 Isolate: For the plaque reduction assay, virus was diluted to infect cells at 50 PFU/well in VGM. 2 hours post infection, plates were washed and overlayed with 2X VGM:Agarose overlay containing serially diluted DAS181 (1000 nM-0.1 nM) or with no DAS181 for control. Viral infection was allowed to continue for 48 hours, and then cells were fixed and stained with the DFA reagent described above. The first plaque reduction assay conducted did not yield accurate EC50 values, in that the dose dependent loss of viral infection demonstrated a lower EC50 value when compared to the graphs in subsequent assays and thus was not included in the average EC50 calculation. It was determined that because the growth properties of this virus are much different than the other strains of PIV3 tested thus far, that this initial experiment was an outlier. Three different plaque reduction assays were conducted after growth properties of the virus were established, and EC50 values for each experiment are indicated (**Figure 15**). Plaque reduction assays were conducted 3 times on three different days to ensure accurate EC50 values across multiple days and passages. The average EC50 value established for this virus is ~28 nM.

DAS 181 Inhibition of TCID50 of the Clinical PIV3 Isolate: Given that viral production was greatly inhibited by DAS181 using the plaque reduction assay, we also tested the ability of the drug to inhibit virus at a higher multiplicity of infection. To accomplish this, cells were infected at the approximate TCID50 identified for the PIV3 clinical isolate, and then were treated with serially diluted DAS181 (0.1-1000 nM) 2 hours post infection and overlayed with agarose. 3 days post infection, cells were fixed and stained with an antibody specific for PIV3 (**Figure 16**). Viral antigen is represented in green. Inhibition of viral infection was observed between 10-100 nM, whereas cells treated with 0.1-1 nM DAS181 exhibited similar viral spread as the no drug control. In support of the plaque reduction assay, these results suggest that the inhibitory concentration of DAS 181 required to limit spread of the viral infection was between 10-100 nM. Formation of pin-point plaques was observed in each of the wells that were infected; however the spread was substantially inhibited at all tested concentrations of DAS181 above 1 nM. In wells with no DAS181 or with 0.1 nM DAS181, the cellular monolayer exhibited significantly enhanced CPE as compared to wells treated with higher concentrations with DAS181, indicating that DAS 181 also has a protective effect in regard to cytotoxicity (data not shown).

DAS 181 Inhibition of Viral Spread and Release: To better quantify the inhibition of viral infection, viral growth analyses were conducted. First, viral spread was monitored throughout the course of infection (72 hours) using DFA and plaque assay to determine viral quantities released into the supernatant. To do this, cells were infected at an MOI of 0.01, and then virus was removed 2 hours post infection and cells were treated with 100 nM DAS181 (or mock-treated with PBS). Every 24 hours, cells were monitored for CPE and viral supernatant was collected and frozen for later titer analyses. At all times post infection, treatment with 100 nM DAS181 protected the cellular monolayer from cytotoxic effects of the viral infection (data not shown). Viral release was also inhibited throughout the course of infection by ~1 log, although by day 3 post infection the viral titers (PFU/mL) released into the supernatant were comparable (**Figure 17**). By day 3 post infection, cells that were mocktreated exhibited approximately 95% cell death, as indicated by the drop in viral titer, whereas wells treated with DAS181 still had approximately 70% of cellular monolayer surviving. These results would explain why the viral titers eventually became comparable in this assay, and is likely due to the fast progression of viral infection for this particular isolate.

### Conclusion

DAS 181 effectively inhibits this clinical strain of PIV3 at all concentrations between 10-1000 nM. The established EC50 was 28 nM. DAS181 treatment over the course of infection in cell culture effectively reduces viral release over time by approximately one log during the active infection cycle. Cytotoxicity and cell death induced by PIV3 infection is substantial by Day 3 post infection when infected at a low MOI and left untreated with DAS181, whereas treatment with DAS181 successfully protects the cellular monolayer from viral induced cytotoxicity. These data extend the current knowledge of the ability of DAS 181 to effectively inhibit different isolates of PIV, and further demonstrates that DAS 181 is effective against clinical isolates of PIV3, even those that are considered the most virulent of strains.

### Materials and Methods

Cells and Viruses: Original LLC-MK2 cells were received from ATCC (Manassas, VA) and have been passaged minimal times (less than 4) to obtain multiple source vials. Cells were thawed prior to receipt of the subject specimens, and passaged at least 2 times before inoculation with the test sample. Specimens containing PIV3 were inoculated into the LLC-MK2 cells, and were passaged 2 times to obtain a large volume of viral supernatant. Viral supernatant was collected that had undergone minimal passages in cell culture in order to maintain the characteristics of the virus.

Cell Culture Maintenance and Viral Growth Medium: Cells were split every 3-4 days, and fed every 2-3 days with Eagles-MEM (Cat. # 11095-098, Life Technologies, Carlsbad, CA), 10% FBS (Cat. # 14-502F, Lonza, Riverside, CA), 1X Glutamax (Cat. # 35050, GIBCO, Carlsbad, CA), and 1X Antibiotic/Antimycotic solution (Cat. # A5955, Sigma, St. Louis, MO). Cells were maintained in ample media according to standard protocols, and were grown at 37°C in a humidified chamber containing 5 % CO₂, unless removed for maintenance or testing. Cells were washed with PBS (Cat. # 14040, GIBCO, Carlsbad, CA), and trypsinized using TrypLE Express (Cat# 12605-010, GIBCO, Carlsbad, CA). Individual flasks of cells were maintained according to standard protocols, and were labeled with the date of passage, initials of scientist, cell passage number, and the name of the cells. Viral infections were performed in the appropriate testing apparatus, including 6 or 24 well plates (Corning, Lowell, MA), as defined by the experiment. Cells were maintained in polystyrene flasks (Corning, Lowell, MA) during amplification before infection. Viral growth media consisted of E-MEM (listed above), 1X Glutamax (listed above), 3.0 mg/mL acetylated trypsin (Cat. # T6763, Sigma) diluted to a final concentration of 3.0 µg/mL, and 1% ITS (Cat. # 41400, GIBCO, Carlsbad, CA). Plaque assay overlay medium consisted of a 1:1 (vol:vol) mixture of the E-MEM media listed above (2X concentration) and 1.8% Noble Agar (Cat. # 10907, USB Corp., Cleveland, OH) in dH20 to achieve a final concentration of 1X media and 0.9% agarose. The lot of DAS181 used for these studies was Lot #20080715, prepared on 20-Jan-09 at a concentration of 25.5 mg/mL

Antibodies and Staining Reagents: For TCID50 plate staining, a Direct Fluorescence Antibody analysis kit was used (Cat. # 3137,Millipore, Temecula, CA) according to manufacturers instructions.

Examples 3-10 below describe the results of a subset of EIND patients treated with DAS 181 using either a nebulizer and a liquid formulation of DAS181 or a dry powder inhaler and a dry formulation of DAS181.

### Example 3 - Treatment of Patient 1 with Nebulized DAS181

Treatment with DAS181 was initiated for an 18 month infant (female) with diagnosed PIV3 infection. This infant was also concomitantly diagnosed with Acute lymphoblastic leukemia (ALL). The initial conservative dosing plan was drafted based on existing animal toxicology data. Due to the age of the patient, the drug could only be delivered in nebulized form, The nebulizer used in this case is described in **Table** 1.

**Table 1: Nebulizer Characteristics**

| | |
|---|---|
| Nebulizer | Aerogen Pro X |
| MMAD | 2.1 µm |
| Fine Particle Fraction (FPF, 1-5 µm) | 68.2% |
| Mean Output | 0.35 mL/min at flow rate of 6 to 60 L/min. |

The initial dosing plan was devised while the patient was intubated. It was advised to start with a 2 min dose based on the available toxicology information. At 0.35 mL/min output, the respirable (1-5 µm) rate was 0.24 mL/min, corresponding to 0.16 mg DAS181/min delivered. For a 2 min dose, 0.32 mg DAS181 respirable aerosol was projected to be delivered (a total of 0.46 mg DAS181 delivered).

### Follow-up dosing plan:

If no symptoms of adverse effect were observed and patient was stable clinically, the duration of nebulization was increased to 4 min, and and symptoms were monitored for the following two days again.

Following the initial three days of dosing, the PIV viral load dropped substantially as measured by quantitative PCR specific for PIV3. The patient was initially determined to have a very high viral load (10⁹ copies/mL in the tracheal aspirate), and this level dropped over 5 logs (to 10⁴ copies/mL) within two days after the last day of dosing. However, the patient had a rebound in viral load after the initial dosing was stopped, indicating the initial doses were not sufficient to clear the infection. During this time, the patient improved clinically, exhibiting slightly clearer chest X-rays. Improvement in ventilator support was also observed following these initial doses of DAS 181. Due to the lack of clearance of the virus as well as the clinical status of the patient, it was recommended to continue dosing the patient with DAS 181.

The patient was dosed again for 4 minutes. Mild clinical improvement was observed, but the patient was still positive for PIV by both qualitative DFA and quantitative PCR assessments. The fifth dose of DAS 181 was given to the patient. The patient's clinical status continued to improve, and the patient was extubated after 5 doses.

Even though there was clinical improvement, the patient's PIV viral load only dropped slightly (~1 log) following the single, intermittent doses of DAS 181. It was recommended to treat the patient with another course of DAS 181. The dosing plan was revised slightly because the patient was extubated as described above.

In non-intubated younger children, a face mask is the easiest way to deliver a nebulized drug in regard to patient compliance, ease of use, and patient comfort. Drug loss to the oral and nasal cavities as well as the delivery efficiency using the face mask was considered in order to estimate proper dosing. A longer dose was required to account for the loss of delivery efficiency.

Development data on DAS181 dry powder with particle size of 3 to 5 µm demonstrated that approximately 30 to 35 % of delivered drug will deposit to oral cavity and oropharynx. Literature data also shows that lung deposition is about 48% of emitted dose when using nebulizer and face mask on children in an ideal situation. It should be noted that the literature concerning drug deposition in the lungs of an infant vary considerably, and are highly dependent on flow rate, infant cooperation, mask fit/design, and dosing time. Taking together the contributing factors of delivery efficiency, at least 50% reduction in delivery efficiency was expected. Based on this, it was recommended to start with 8 minutes of dosing using the standard nebulizer with the face mask setup. The additional dosing time accounted for potential loss to oral/nasal cavity and face mask setting compared to dosing the patient while intubated. There was no change in dosing solution preparation. For an 8 minute dose, 1. 8 mg total DAS181 was expected to be delivered, and 1.25 mg DAS 181 was expected to be in respirable form.

The patient was treated with five (8 minute) once daily doses of DAS 181 Following this round of dosing, the PIV3 viral load again dropped substantially (greater than 3 logs) as demonstrated by quantitative assessment of viral load in nasal wash samples taken from the patient immediately prior to each dose, and in the days following dosing. The reduction in viral load continued to drop for 5 days following the last dose as shown in **Figure 18** (dosing days are indicated in red), and continued to decline well after dosing. The patient was eventually discharged from the hospital and the viral load dropped to undetectable.

The results from this case demonstarate a viable delivery method for nebulized DAS 181 solution to both a patient that is intubated and a patient using face mask. The estimated dosing plan was accurate, and a comparable amount of DAS181 was delivered with both methods. In addition, these data support the use of DAS181 in young infants, demonstrating safety, effective drug delivery, and antiviral effects of the drug when used with this delivery method.

### Example 4 - Treatment of Patient 2 with Nebulized DAS181

The patient was a 61 year old male that had a peripheral blood stem cell transplant for AML. The post transplant course was complicated by skin GVHD, RSV pneumonia, lung nodules of uncertain etiology and an episode of bladder symptoms thought to be due to GVHD, for which he was treated with steroids. The following year he presented to an outside hospital with cough, dyspnea and chest pain. He underwent a BAL, was started on Cidofovir for possible adenovirus pneumonia, and was transferred a hospital on July 1 with progressive respiratory failure. He was found to have blood adenovirus (86,000 copies/ml) and had adenovirus (Ct=38.4) and PIV-3 (Ct=32.3) in a nasopharyngeal sample by PCR. Cidofovir was continued (1 mg/kg, 3x/week). He was intubated at which time a BAL showed no adenovirus or other pathogens but was positive for PIV-3 by DFA and by PCR (Ct=18.8). His condition was gradually deteriorating with increasing need for ventilator support (FIO2=~90%, 10 mm PEEP) prior to treatment. A tracheal aspirate was positive for PIV3 with a Ct = 13, presumably a very high viral load.

DAS181 was to be given via in-line nebulizer once daily. The concentration of DAS181 in the solution was to be 1.29 mg/mL. On day 1, 1.5 ml was to be delivered, while on day 2, the dose was to be increased to 2.5 ml. On days 3-5 the recommended dose was between 2.5 ml-5 ml with the final dose chosen based on the patient's clinical response. Daily viral loads, laboratory analyses, and clinical observations were to be conducted.

After one day of treatment of nebulized DAS 181 (1.5 mL), the patient tolerated the drug well, and the viral load dropped approximately 1 log. At an output rate of 0.35 mL/min, this amount was dosed in approximately 4 min. The dose generated was approximately 1.3 mg DAS181 in respirable range.

After the second dose (2.5 mL, 2.2 mg DAS181 in the respirable range), the patient remained alive and was documented to be slightly better in that his oxygenation was slightly improved (0.9 FiO2 and 10 of PEEP with sats of 93% instead of 1.0 and 12 with sats of 88-90%) and his lung compliance improved (tidal volumes of 430 on 18 pressure control as opposed to 380 on 20 pressure control). His chest radiograph continued to have diffuse infiltrates but may have been less dense in the upper lungs bilaterally. The viral load dropped greater than one log after two doses as shown below in Table 2.

**Table 2**

| **Dosing Day** | **Viral Load (viral RNA copies/mL)** |
|---|---|
| Pre-treatment | 6.27 × 10¹⁰ |
| Day 1 post-dose | 9.41 × 10⁹ |
| Day 2 post-dose | 1.96 × 10⁹ |

Following the second dose of DAS181, the patient's family decided that they wished to withdraw all life-sustaining measures. Thus, no additional doses of DAS 181 were administered.

### Example 5 - Treatment of Patient 3 with Nebulized DAS181

This patient was a 47 year old female who was evaluated for possible interstitial lung disease. This patient was diagnosed with possible hypersensitivity pneumonitis, and was treated with steroids. The patient was admitted with respiratory failure. A BAL collected from this patient was positive for PIV-3 by qualitative PCR (respiratory viral panel). All other diagnostic tests were negative. Diffuse pneumonitis was observed in this patient, and she was on ECMO for oxygenation.

The proposed dosing for this patient was administration of DAS 181 via nebulizer due to the patient's deteriorating lung function status. The first dose was to be 1.5 mL of DAS181 stock solution of 1.3 mg/mL concentration, for a total dose of 1.3 mg DAS181 in the respirable range. The second dose was to be increased to 2.5 mL based on the patient's status and laboratory read-outs (2.5 mL of the stock solution equates to 2.2 mg of DAS181 in the respirable range). Dosing between days 3-5 was to be between 2.5-5.0 mL. The Aerogen Pro-X nebulizer system was to be used according to manufacturer's instructions, and the recommendation of the clinical site.

Following 5 days of dosing with DAS181, the patient remained on ECMO for much of the treatment course. Her chest X-ray appeared improved after the first 3 doses. It was suspected that some acute respiratory distress syndrome (ARDS) was occurring, and it was concluded that multiple factors were contributing to her poor lung status. After completion of the treatment course, the patient was removed from ECMO and seemed to able to breathe using supplemental oxygen by face mask, a marked improvement in patient clinical status.

Table 3 summarizes laboratory results obtained from this patient. Virology results were from throat swab samples collected from the subject immediately prior to dosing each day (2 swabs inoculated into 3 mL of standard Copan viral transport medium).

**Table 3**

| **Dosing Day** | **Dose Received** | **PIV3 Viral Load** |
|---|---|---|
| Day 1 | 1.5 mL | 8100 |
| Day 2 | 2.5 mL | 20100 |
| Day 3 | 2.5 mL | 6350 |
| Day 4 | 2.5 mL | BLQ |
| Day 5 | 2.5 mL | BLQ |
| Day 6 (1 day Post Dose) | None | Not Tested |

Figure 19 depicts the data presented in the above table.

### Example 6 - Treatment of Patient 4 with Nebulized DAS181

Patient 4 was a 7 month old male with an underlying disease of SCIDS (T-/B+NK-) complicated with GVHD post bone marrow transplant, who presented with persistent PIV3 infection. The PIV3 infection persisted for approximately 6 weeks prior to DAS181 treatment, and the patient had persistent oxygen requirement throughout. The patient progressed to require mechanical ventilation, and was also diagnosed with pneumonia, which was treated with a 21 day treatment course of steroids and antibiotics. The patient received IVIG, but parainfluenza infection persisted. The patient was extubated, although remained persistently hypoxic with abnormal chest X-rays, requiring persistent oxygen supplementation. The patient received an autologous bone marrow transplant, and became increasingly ill after receiving immunosuppression for GVHD following the bone marrow transplant. PIV3 infection was confirmed prior to treatment with DAS181 by respiratory film array PCR. Additionally, PIV3 was confirmed by direct fluorescence analysis (DFA).

An initial 5 day course of dissolved DAS 181 dry powder delivered via nebulizer was recommended, with the option for a follow-up treatment course of an additional 5 days of dosing. FDA approval for this EIND was granted. The drug was administered via facemask with an Aeroneb nebulizer.

The first dose of DAS181 (1.5 mL; 1.9 mg DAS 18 1) was administered without any adverse effects. Subsequently, the next four doses were administered (1.5 mL; 1.9 mg DAS 181). During this first five days of dosing, the patient began to show modest signs of clinical improvement. Crackles in the lungs were present, but resolved by the time 3^{rd} dose of DAS 181 was given. However, the patient remained symptomatic throughout the first five days of dosing, with continued need for supplemental oxygen (4-6 L via high flow nasal cannula). No adverse effects related to study drug were observed throughout the treatment course. The physicians recommended continued treatment with DAS 181 for an additional round of dosing.

An additional four doses of DAS181 (1.5 mL, 1.9 mg DAS 18 1) were administered via facemask. Due to increases in alkaline phosphatase levels, DAS 181 was administered every other day during the last 3 doses of DAS 181 treatment. During this treatment course, the patient appeared to exhibit substantial clinical improvement. The supplemental oxygen requirements began to improve, dropping to only 0.5 L/min by the end of treatment. The lung function was also reported as substantially improved, both by chest X-ray and by general observation. The patient also experienced a reduction in coughing during the treatment course and the breathing patterns of the patient became more normal.

Viral load results for this patient were obtained from assessment by DFA (semiquantitative) and by qPCR (quantitative). Nasopharyngeal samples were to be tested daily by qPCR, and as needed by DFA. Table 4, below, summarizes the viral load results obtained from each assessment. The DFA readout is measured between negative (no infection observed) to 4+ (100% of cells in the field being positive for PIV). The qPCR result measures RNA copies/mL. Nasopharyngeal swabs were used for both assessments.

**Table 4**

| **Date** | **DFA Result** | **qPCR Result** |
|---|---|---|
| Day1 (baseline (pre-dose 1)) | 4+ | 5.44 × 10⁵ |
| Day 2 (pre-dose 2) | No Data | 6.61 × 10⁶ |
| Day 3 (pre-dose 3) | No Data | 6.14 × 10⁷ |
| Day 4 (pre-dose 4) | 2-3+ | 8.48 × 10⁷ |
| Day 5 (pre-dose 5) | No Data | 7.53 × 10⁷ |
| Day 6 (pre-dose 6) | No Data | 7.38 × 10⁶ |
| Day 7 (pre-dose 7) | 2-3+ | 1.40 × 10⁸ |
| Day 8 (pre-dose 8) | 1+ | 7.50 × 10⁶ |
| Day 9 (no dose administered) | Trace to 1+ | 3.00 × 10⁷ |
| Day 10 (pre-dose 9) | 1+ | 4.93 × 10⁶ |
| Day 12 (no dose administered) | Rare/Trace | 1.03 × 10⁸ |
| Day 14 (no dose administered) | Negative | No Data |

Overall, the patient exhibited marked clinical improvement throughout treatment, with no reported adverse effects of the drug, other than noted increase in alkaline phosphatase. The virological results were somewhat divergent, in that the DFA assessment showed a substantial reduction in viral infection, leading to a negative DFA result by the end of treatment. However, the qPCR results indicate that virus was still present in the samples at the end of the treatment course. It is unknown at this time why the results are different. It is possible that the discrepant results are due to the fact that the DFA assessment measures actively infected in-tact cells, while the PCR measures all viral RNA present in the sample, whether infectious or not.

### Example 7: Treatment of Patient 5 with dry powder DAS181

Patient 5 was a 59 year old man with a history of Crohn's disease, diabetes mellitus and interstitial lung disease who underwent left lung transplantation. He was maintained on a chronic maintenance immunosuppression regimen of tacrolimus, mycophenolate mofetil, and prednisone 5mg daily, in addition to monthly adalimumab therapy for his severe Crohn's disease. His post-transplant course was complicated by bronchomalacia and several respiratory tract infections, including respiratory syncytial virus (RSV) pneumonitis and *Klebsiella* pneumonia. He returned almost to his baseline after these respiratory infections.

He developed fevers, chills, and purulent sputum leading to hospitalization. He defervesced and had resolution of his purulent sputum with empiric therapy with vancomycin, ceftazidime, and levafloxacin; however, his dyspnea on exertion, wheezing, and cough were slow to improve. He was afebrile but had a 2 liter oxygen requirement and wheezing and crackles on lung exam. A chest computed tomography scan was performed which showed inflammatory-appearing infiltrates in his left lower lobe. He continued to receive vancomycin, ceftazidime, and levofloxacin. He had a bronchoscopy and was noted to have yellowish secretions. His bronchoalveolar lavage (BAL) specimen tested positive for parainfluenza-3 (PIV3) by qualitative PCR; all other cultures and viral studies were unrevealing.

The proposed dosing regimen of 10 mg DAS181 for 3-5 days, depending on response and adverse effects. The drug was to be administered via dry powder inhaler, and an additional treatment course could be warranted based on symptomology and safety. Nasopharyngeal swabs were to be collected daily to determine PIV-3 quantitative viral load. Daily laboratories (including complete blood count, comprehensive chemistries, liver function tests, PT, and PTT) were also to be conducted. Baseline and daily pulmonary function tests were also to be conducted.

Vital signs obtained immediately prior to treatment indicated that the patient required 2 liters of supplemental O₂ by nasal cannula. Pulmonary function tests obtained before the administration of his first dose of DAS181 demonstrated a FEV1 of 1.52 liters and FVC of 1.70 liters.

The following samples were collected before each dose: a nasopharyngeal (N/P) swab and oropharyngeal (OP) wash for monitoring of viral shedding, and blood samples for testing of DAS 181 levels. The In-Check DIAL was used for inhalation training. After further satisfactory training in the inhalation technique with the Cyclohaler and an empty capsule, DAS181 was administered via the Cyclohaler. The patient had no immediate reactions to the administration of DAS181.

The patient received treatment for 5 consecutive days without experiencing any evident adverse events. He improved clinically during the treatment course from a respiratory and systemic standpoint. By day 2 of treatment he felt less dyspneic and his cough became dryer. By the last day of treatment, he felt back to about 90% of his baseline in terms of his energy and breathing. On day 6 after starting DAS181, a bronchoscopy was performed and the BAL fluid was again positive for PIV3, with a viral load of 3.50E+07.

The patient was discharged home two days after completing DAS181 treatment. His vital signs post treatment all showed improvement, and when contacted by phone 2 weeks post treatment he reported feeling well with no signs of relapse. His exercise tolerance had returned to his baseline level prior to his illness. Overall, the patient also improved in regard to his oxygen requirement upon completion of the treatment.

Nasal pharyngeal swabs and oropharyngeal wash samples were sent for PIV3 viral load testing. Results are summarized in the following table:

| **Day post dose** | **NP swab PIV copies/ml** | **OP wash PIV copies/ml** |
|---|---|---|
| Day 1 (pre-dose 1) | 7.87 × 10⁵ | 5.39 × 10⁴ |
| Day 2(pre-dose 2) | 1.46 × 10⁵ | 2.63 × 10⁵ |
| Day 3(pre-dose 3) | 7.77 × 10⁶ | 3.46 × 10⁴ |
| Day 4(pre-dose 4) | 8.87 × 10⁷ | 2.67 × 10⁴ |
| Day 5(pre-dose 5) | 1.45 × 10⁵ | 1.73 × 10⁶ |
| Day 6 (+1) (1 day post dose 5) | 2.01 × 10⁴ | 4.41 × 10⁵ |
| Day 7 (+2) (2 days post dose 5) | 2.56 × 10⁴ | 2.20 × 10³ |

There was some day-to-day variability in quantitative PIV3 viral load measurements, possibly due to differences in sample collection techniques by different providers and the exquisite sensitivity of the assay itself to small variations in virions in the sample. The data suggest ≥ 1log drop in viral load irrespective of the sample type.

### Example 8: Treatment of Patient 6 with dry powder DAS181

Patient 6 was a 51-year-old woman with a history of breast cancer and treatment-related AML s/p allogeneic HSCT. Despite remission of her leukemia, she had developed chronic graft-versus-host disease and bronchiolitis obliterans syndrome requiring treatment with mycophenolate mofetil, imatinib, and chronic steroids. She developed an acute increase in her dyspnea to the point where she was unable to perform her basic activities of daily living. She also developed a new fever and persistent nonproductive cough. She was admitted to the hospital for further care. Chest CT demonstrated diffuse ground glass opacities and some bronchovascular nodular opacities. PCR of an admission nasopharyngeal swab was positive for parainfluenza 1 (PIV1) and negative for influenza and RSV. Bronchoalveolar lavage was performed and PCR for PIV1 was again positive. She had a persistent dry cough, dyspnea on exertion, and a 2L supplemental oxygen requirement.

The proposed dosing regimen was 10 mg of DAS181 delivered daily via dry powder inhaler for up to 5 days depending on response and if adverse effects were noted. It was planned to obtain nasopharyngeal swab samples for determination of quantitative parainfluenza virus PCR and viral cultures. Safety laboratories including complete blood count, and comprehensive chemistries were to be collected. Baseline and daily pulmonary function tests while on therapy were to be conducted. An additional 5 doses of DAS181 was left as a possibility, pending the patient's symptomology and safety.

Samples were collected before each dose including: nasopharyngeal (NP) swabs, oropharyngeal wash (OP), and blood DAS 181 PK samples. The In-Check DIAL was used for inhalation training. Pulmonary function test (PFTs) were also performed. On day 1 of treatment results were: forced expiratory volume in 1 second (FEV 1)=0.78; forced vital capacity (FVC)=1.78. After further training of the inhalation technique with the Cyclohaler and an empty capsule that was considered satisfactory, the clinical site proceeded to administer the DAS181 capsule via the Cyclohaler. The patient required 3 inhalations to empty the contents of the capsule. She had no immediate reactions to the administration.

The patient received treatment for 5 consecutive days, without experiencing any evident adverse events. She improved clinically during the treatment course. By day 2 of treatment, she was discharged home with less subjective shortness of breath. She self administered DAS 181 treatment on days 3-4. By the last day of treatment, she felt much better with slightly increased exercise tolerance, but not yet back to her respiratory baseline. Secretions and cough decreased. PFTs on the last day of treatment showed a FEV1 of 0.83 L and FVC of 1.96 L. She was evaluated 4 days after her last dose and her symptoms improved even further. The physician called the patient days later and she noted that she was feeling well, without any adverse effects related to the drug. Her shortness of breath was substantially improved, and she was able to reduce her steroid dose for treatment of her chronic pulmonary graft-versus-host disease and bronchiolitis obliterans syndrome.

Nasopharyngeal and oropharyngeal samples were sent for PIV-1 viral load testing, with the following results.

| **Day Post Dose** | **N/P swab PIV1 copies/ml** | **O/P wash PIV1 copies/ml** |
|---|---|---|
| Day 1 (pre dose 1) | 9.60 × 10⁵ | 3.95 × 10⁴ |
| Day 2 (pre dose 2) | 5.95 × 10⁵ | 4.82 × 10⁵ |
| Day 5 (pre dose 5) | 3.42 × 10⁷ | 5.30 × 10⁶ |
| Day 9 (4 days post dose 5) | 1.03 × 10⁴ | 5.70 × 10³ |

Both the nasopharyngeal and oropharyngeal PIV viral loads showed substantial drop following treatment with DAS181, which paralleled with her marked clinical improvement.

### Example 9: Treatment of Patient 7 with dry powder DAS181

Patient 7 was a 64 year old female with a history of idiopathic pulmonary fibrosis (IPF) who underwent right lung transplant. Her initial post-transplant course was complicated by acute humoral rejection managed with plasmapheresis and IVIG and was unable to tolerate MMF or Imuran so was maintained on prednisone and tacrolimus. Several weeks prior to admission her husband became ill with an upper respiratory infection from which he uneventfully recovered. The patient then began to experience increasing shortness of breath and cough, took a home O₂ saturation which was 80%, and was subsequently admitted to the clinical institution. A bronchoscopy (BAL) showed no evidence of bacterial or fungal infection, or PJP, but did return positive for PIV3 by PCR. Later, she developed worsening hypoxia and was transferred to the intensive care unit for high-flow oxygen support and monitoring. She continued to require high-flow oxygen support and remained at an FiO₂ of 65% without evidence of improvement. Her immunosuppression was being minimized to the extent possible.

Dosing with DAS181 was initiated. The patient received 5 doses of DAS 181 (10 mg/day via dry powder inhaler). The patient received the drug and responded quite well, with rapid improvement in both virologic and clinical parameters. She had no significant adverse effects associated with the drug. Marked improvement in PIV3 viral load was observed, and is shown in Figure 20.

An overview of dosing, concomitant relevant medications, viral load, and supportive oxygen requirements is shown in Figure 21. As can been seen, the viral load and required oxygen support were markedly reduced following dosing with DAS181.

### Example 10: Treatment of Patient 8 with dry powder DAS181

Patient 8 was a 57 year old man with a history of Hodgkin's disease who received allogeneic HSCT for recurrent disease. He later relapsed and received donor lymphocyte infusion. His clinical course was complicated by graft-versus-host disease (GVHD).

He was admitted with complaints of shortness of breath, cough, hemoptysis and new onset nephrotic syndrome. Bronchoscopy with bronchoalveolar lavage was significant for diffuse alveolar hemorrhage. CT scan of the chest was notable for diffuse ground glass opacities, and the patient was confirmed parainfluenza type 3 by PCR on BAL fluid. His clinical status was worsening and he was requiring 5 Liters/minute oxygen (O₂) and was saturating at 93%. His oxygen saturation dropped in the 80's with minimal activity.

The use of DAS181 in this patient's case was approved by FDA. The approved dosing regimen was administration of DAS 181 dry powder for 5 consecutive days. Nasopharyngeal swab samples were collected before each dose to assess viral load. After training of the inhalation technique with the Cyclohaler that was satisfactory, DAS 181 was administered. The patient had no immediate reactions to the administration. He received treatment for 5 consecutive days, without experiencing any evident adverse events. The patient took his last dose of DAS 181 on and was discharged from the hospital after improving clinically.

Nasopharyngeal samples were assessed for PIV3 RNA copies/mL and showed a substantial drop in viral load, eventually leading to undetectable titers.

| **Day of Dosing (Day 1=Dose 1)** | **Nasopharyngeal Swab PIV3 copies/mL** |
|---|---|
| Day 1 (sample taken pre-dose 1) | 26,400 |
| Day 2 (sample taken pre-dose 2) | 4,900 |
| Day 3 (sample taken pre-dose 3) | 11,600 |
| Day 4 (sample taken pre-dose 4) | 3,290 |
| Day 5 (sample taken pre-dose 5) | Undetectable |

As can be seen in the table above, the viral load dropped to undetectable following treatment with DAS 181 dry powder for 5 days. This drop in viral load also correlated with the clinical improvement and subsequent discharge from the hospital experienced by this patient. The patient also required substantial supplemental oxygen support prior to treatment with DAS 181, which was alleviated following the treatment.

### Example 11 - Preparation of DAS181

### Preparation of DAS181 for use in Aerosol Formulations

A DAS181 (1.0 -10.0 mg/mL) stock solution in water can be stored at 2 - 8°C for at least one week. Dose solutions at lower concentration are prepared fresh daily and stored at ambient conditions or refrigerated until use. For dose solutions, the stock solution can be diluted in normal saline or other pharmaceutically suitable aqueous solution.

DAS181 is a fusion protein containing the heparin (glysosaminoglycan, or GAG) binding domain from human amphiregulin fused *via* its N-terminus to the C-terminus of a catalytic domain of *Actinomyces Viscosus* (sequence of amino acids in DAS 181 having an amino terminal Met is set forth in SEQ ID NO: 1; sequence of amino acids in DAS181 lacking an amino terminal Met is set forth in SEQ ID NO: 1).

DAS 181 protein can be prepared and purified as described in Malakhov et al. 2007 Antimicrobial Agents Chemotherapy 1470-1479. Briefly, a DNA fragment coding for DAS181 with an amino terminal Met was cloned into the plasmid vector pTrc99a (Pharmacia) under the control of a IPTG (isopropyl-β-D-thiogalactopyranoside)-inducible promoter. The resulting construct was expressed in the BL21 strain of *Escherichia Coli* (*E.Coli).* The *E*. *coli* cells expressing the DAS181 protein were washed by diafiltration in a fermentation harvest wash step using Toyopearl buffer 1, UFP-500-E55 hollow fiber cartridge (GE Healthcare) and a Watson-Marlow peristaltic pump. The recombinant DAS 181 protein can be purified in bulk from the cells as described in published US 2005/0004020 and US 2008/0075708.
SEQ ID NO:1 
MGDHPQATPA PAPDASTELP ASMSQAQHLA ANTATDNYRI PAITTAPNGD
LLISYDERPK DNGNGGSDAP NPNHIVQRRS TDGGKTWSAP TYIHQGTETG
KKVGYSDPSY VVDHQTGTIF NFHVKSYDQG WGGSRGGTDP ENRGIIQAEV
STSTDNGWTW THRTITADIT KDKPWTARFA ASGQGIQIQH GPHAGRLVQQ
YTIRTAGGAV QAVSVYSDDH GKTWQAGTPI GTGMDENKVV ELSDGSLMLN
SRASDGSGFR KVAHSTDGGQ TWSEPVSDKN LPDSVDNAQI IRAFPNAAPD
 
DPRAKVLLLS HSPNPRPWSR DRGTISMSCD DGASWTTSKV FHEPFVGYTT
IAVQSDGSIG LLSEDAHNGA DYGGIWYRNF TMNWLGEQCG QKPAKRKKKG
GKNGKNRRNR KKKNP
SEQ ID NO:2

GDHPQATPAP APDASTELPA SMSQAQHLAA NTATDNYRIP AITTAPNGDL
LISYDERPKD NGNGGSDAPN PNHIVQRRST DGGKTWSAPT YIHQGTETGK
KVGYSDPSYV VDHQTGTIFN FHVKSYDQGW GGSRGGTDPE NRGIIQAEVS
TSTDNGWTWT HRTITADITK DKPWTARFAA SGQGIQIQHG PHAGRLVQQY
TIRTAGGAVQ AVSVYSDDHG KTWQAGTPIG TGMDENKVVE LSDGSLMLNS
RASDGSGFRK VAHSTDGGQT WSEPVSDKNL PDSVDNAQII RAFPNAAPDD
PRAKVLLLSH SPNPRPWSRD RGTISMSCDD GASWTTSKVF HEPFVGYTTI
AVQSDGSIGL LSEDAHNGAD YGGIWYRNFT MNWLGEQCGQ KPAKRKKKGG
KNGKNRRNRK KKNP

## Claims

1. A composition comprising a therapeutically effective amount of a protein comprising SEQ ID NO: 1 or SEQ ID NO: 2 for use in a method of treating parainfluenza or influenza virus infection, the method comprising administering said composition to the respiratory tract of a patient, wherein the composition is in liquid form and the administration is by nebulizer, wherein 0.1 - 10mg of the protein is administered to the patient per day.

2. The composition for use according to claim 1 wherein the patient is an immunocompromised patient.

3. The composition for use according to claim 2 wherein the immunocompromised patient is suffering from a primary immunodeficiency.

4. The composition for use according to claim 2 wherein the immunocompromised patient is suffering from a secondary immunodeficiency.

5. The composition for use according to claim 2 wherein the immunocompromised patient is being or has been treated with an immunosuppressive therapy.

6. The composition for use according to claim 2 wherein the immunocompromised patient is being or has been treated with a chemotherapeutic agent.

## Patentansprüche

1. Zusammensetzung, umfassend eine therapeutisch wirksame Menge eines Proteins, das SEQ ID NO: 1 oder SEQ ID NO: 2 umfasst, zur Verwendung bei einem Verfahren zur Behandlung einer Parainfluenza- oder Influenzavirusinfektion, wobei das Verfahren Verabreichen der Zusammensetzung an die Atemwege eines Patienten umfasst, wobei die Zusammensetzung in flüssiger Form vorliegt und die Verabreichung mit einem Vernebler erfolgt, wobei dem Patienten pro Tag 0,1 - 10 mg des Proteins verabreicht werden.

2. Zusammensetzung zur Verwendung nach Anspruch 1, wobei es sich bei dem Patienten um einen immungeschwächten Patienten handelt.

3. Zusammensetzung zur Verwendung nach Anspruch 2, wobei der immungeschwächte Patient an einer primären Immunschwäche leidet.

4. Zusammensetzung zur Verwendung nach Anspruch 2, wobei der immungeschwächte Patient an einer sekundären Immunschwäche leidet.

5. Zusammensetzung zur Verwendung nach Anspruch 2, wobei der immungeschwächte Patient mit einer Immunsuppressionstherapie behandelt wird oder wurde.

6. Zusammensetzung zur Verwendung nach Anspruch 2, wobei der immungeschwächte Patient mit einem Chemotherapeutikum behandelt wird oder wurde.

## Revendications

1. Composition comprenant une quantité thérapeutiquement efficace d'une protéine comprenant la SEQ ID NO: 1 ou la SEQ ID NO: 2 pour une utilisation dans un procédé de traitement d'une infection par un virus de parainfluenza ou de la grippe, le procédé comprenant une administration de ladite composition aux voies respiratoires d'un patient, la composition étant sous forme liquide et l'administration étant par un nébuliseur, 0,1 à 10 mg de la protéine étant administrée au patient par jour.

2. Composition pour une utilisation selon la revendication 1, le patient étant un patient immunocompromis.

3. Composition pour une utilisation selon la revendication 2, le patient immunocompromis souffrant d'une immunodéficience primaire.

4. Composition pour une utilisation selon la revendication 2, le patient immunocompromis souffrant d'une immunodéficience secondaire.

5. Composition pour une utilisation selon la revendication 2, le patient immunocompromis étant traité ou ayant été traité avec une thérapie immunosuppressive.

6. Composition pour une utilisation selon la revendication 2, le patient immunocompromis étant traité ou ayant été traité avec un agent chimiothérapeutique.
